Europäisches Patentamt

**European Patent Office**

**Office européen des brevets**

(11) Publication number: **0 386 304**
**A1**

# EUROPEAN PATENT APPLICATION

(21) Application number: **89109656.2**

(22) Date of filing: **29.05.89**

(51) Int. Cl.⁵: **C12N 15/12, C12P 21/02,**
**C12N 5/20, C12P 21/08,**
**//(C12P21/08,C12R1:91)**

The microorganism(s) has (have) been deposited with Fermentation Research Institute under number(s) FERM BP-2312, 2313, 2314, 2315, 2435.

The title of the invention has been amended (Guidelines for Examination in the EPO, A-III, 7.3).

Claims 11-15 are is deemed to be abandoned due to non-payment of the claims fees (Rule 31 (2) EPC).

(30) Priority: **07.03.89 EP 89104023**

(43) Date of publication of application:
**12.09.90 Bulletin 90/37**

(84) Designated Contracting States:
**GB**

(71) Applicant: **Taniguchi, Tadatsugu, Dr.**
**Institute for Molecular and Cellular Biology**
**Osaka University 1-3, Yamadaoka**
**Suita-shi Osaka 565(JP)**

Applicant: **Miyasaka, Masayuki, Dr.**
**Department of Immunology The Tokyo**
**Metropolitan Institute of Medical Science**
**Bunkyo-ku Tokyo 113(JP)**

(72) Inventor: **Taniguchi, Tadatsugu Dr**
**Inst Molecular and Cellular Biology**
**Osaka University 1-3,Yamadaoka**
**Suita-shi(JP)**
Inventor: **Kono, Takeshi**
**1-8-20-301, Teshinakita**
**Ikeda-shi Osaka 563(JP)**

(74) Representative: **Laudien, Dieter, Dr. et al**
**Boehringer Ingelheim Zentrale GmbH ZA**
**Patente Postfach 200**
**D-6507 Ingelheim am Rhein(DE)**

(54) **Recombinant interleukin-2 receptor.**

(57) Recombinant IL-21Rβ chain or portions thereof, cDNA coding therefore, vectors containing said cDNA, hosts transfected by said vectors, and monoclonal antibodies to said recombinant IL-2Rβ or portions thereof.

EP 0 386 304 A1

Xerox Copy Centre

## Recombinant Protein Receptor

This invention relates to receptors for interleukin-2, more particularly to the $\beta$-chain of the receptor, and to cDNA coding for the $\beta$-chain or parts thereof, vectors containing cDNA inserts coding for the $\beta$-chain, hosts transformed by such vectors and the cultivation of such hosts to produce the said $\beta$-chain.

Ample evidence has been accumulated that cytokines, a class of soluble mediators involved in cell-to-cell "communications", are essential in the regulation of the immune system. It has been known that cytokines induce proliferation, differentiation and activation of target cells through interaction with specific cell surface receptor(s). Interleukin-2 (IL-2), previously defined as T cell growth factor (1), is one of the best characterized cytokines, known to play a pivotal role in the antigen-specific clonal proliferation of T lymphocytes (T cells) (2). IL-2 also appears to act on other cells of the immune system such as immature thymocytes (3), B lymphocytes (B cells) (4), macrophages (5), natural killer cells (NK cells) (6), and lymphokine-activated killer cells (LAK cells) (7). These multifunctional properties of IL-2 have now opened up possibilities in the formulation of immunotherapies such as adoptive immunotherapy (8). More recently, IL-2 has been shown to function also on neural cells such as oligodendrocytes (9), suggesting a possible involvement of this cytokine in the central nervous system. Despite extensive studies on the IL-2 system in the context of basic and clinical immunology, information has been limited on the molecular mechanism(s) underlying the IL-2-mediated signal transduction (10).

The IL-2 receptor (IL 2R) is known to be unique in that it is present in three forms: high-, intermediate- and low-affinity forms with respect to its binding ability to IL-2, and respective dissociation constants (Kds) of $10^{-11}M$, $10^{-9}M$ and $10^{-8}M$ (11, 12). Following the characterization of IL-2R$\alpha$ chain (Tac antigen, p55) (13), it became evident that the $\alpha$ chain constitutes solely the low-affinity form and it is not functional per se in IL-2 internalization and signal transduction, unless associated with another specific membrane component(s) of lymphoid cells (14, 15). Subsequently, the lymphoid membrane component was identified to be a novel receptor chain, termed $\beta$ chain (or p70-75) (12, 16, 17). In fact, experimental evidence has suggested that the IL-2R$\beta$ chain per se constitutes the intermediate-affinity form (12). In addition, its association with the IL-2R$\alpha$ chain results in the high-affinity form of the receptor (12, 16, 17). Expression studies using wild type and mutated IL-2R$\alpha$ chain cDNAs strongly support the notion that the IL-2R$\beta$ chain but not the IL-2R$\alpha$ chain possesses a domain(s) responsible in driving the intracellular signal transduction pathway(s) (18). There exists therefore a need to obtain IL-2$\beta$ chain in amounts which will enable its structure and function to be elucidated, this being an essential step in gaining further insight into the molecular basis of the high-affinity IL-2R as well as on the mechanism of signal transduction operating in IL-2 responsive cells. To this end we describe below cDNA coding for the IL-2R$\beta$ chain or parts thereof whereby insertion of said cDNA in a suitable vector and expression thereof in an appropriate host will enable recombinant and large scale production of protein corresponding to the IL-2R$\beta$ chain or parts thereof.

### Isolation and analysis of the cDNA clones coding for human IL-2R$\beta$ chain

In isolating the cDNA clones, we applied an expression cloning strategy by using the monoclonal antibodies, Mik-$\beta$1 and Mik-$\beta$2 (19), both of which have been raised against the IL-2R$\beta$ chain found on the human leukemic cell line (YT (20). The monoclonal antibodies Mik-$\beta$1 and Mik-$\beta$2 are both deposited at Fermentation Research Institute, Agency of Industrial Science and Technology, Japan. The deposit numbers for Mik-$\beta$1 and Mik-$\beta$2 are, 10453 and 10454 (1988), respectively; they are also described in Japanese Patent Application No. 298742 (1988).

A few sets of cDNA libraries were prepared by using the poly(A)$^+$-RNA from YT cells according to standard procedures. cDNA libraries were prepared with cDM8 vector according to published procedures (21), except using random primer (Amersham) or oligo (dT) primer as mentioned below. The plasmid DNA representing $5.6 \times 10^6$ independent colonies were prepared by the standard procedure and one mg of DNA were used for the first DNA transfection. Actually, the DNA was divided into 100 tubes (therefore each tube contained 10 $\mu$g of DNA) and they were each transfected into $3.5 \times 10^5$ monkey COS cells in a tissue culture dish (60 mm polystyrene dish, Corning). The transfection was done using the standard DEAE dextran procedures. The transfected COS cells were then treated with the cocktail of Mik-$\beta$1 and -$\beta$2 antibodies (400-fold diluted ascites for each antibody) and subjected to the standard panning procedure. The dish used for the panning was FALCON 60 mm dish, coated with anti-mouse IgG as described previously (ref.

21). In this first round of panning, 100 IgG-coated dishes were used. After the panning, Hirt extract was prepared by the standard procedure (ref. 21) and the recovered plasmids were introduced into E.coli by the method described in ref. 21. By this procedure $3.7 \times 10^6$ colonies were obtained. Those bacterial colonies were fused with COS cells by the standard protoplast fusion procedures (ref. 21). In these fusion experiments, 26 Corning dishes each containing $5 \times 10^5$ COS cells were used. After the fusion, the COS cells were subjected to panning as described above and Hirt extract was prepared. 32,000 bacterial colonies were obtained from the Hirt extract. The fusion, panning procedures were repeated again and 32,000 bacterial colonies were obtained from the subsequent Hirt extract. The same procedure were repeated once again, obtaining 28,000 bacterial colonies (in the meantime, there should be a dramatic enrichment of the objective clones). The same procedures were repeated once again and 6,000 colonies were obtained. From these colonies, 30 colonies were picked up randomly and the cDNA inserts were analysed. Of them, only 7 colonies contained plasmids from which cDNA inserts can be excised by restriction enzyme XhoI. The vector derived XhoI sites are located at the both side of the cDNA and all other plasmids had lost such cleavage sites due to the DNA rearrangements; in fact, all of them were much smaller in size than the original vector. Thus they were considered to be non-specific products. On the other hand, all of the 7 colonies were derived from the same mRNA, as confirmed by the conventional restriction enzyme cleavage analysis and DNA blot analysis. Of them, one plasmid, termed pIL-2R$\beta$30 contained longer cDNA than the other 6 plasmids which turned out to be identical to each other (designated gTT-2R$\beta$9)

In this procedure, therefore, we isolated two independent cDNA clones, pIL-2R$\beta$9 and pIL-2$\beta$30; each of the expression products specifically reacted with the antibodies. The two clones contained cDNA inserts of 1.3Kb and 2.3Kb, respectively, and cross-hybridized with each other. Subsequent sequence analysis of the cDNAs revealed that they represent the same mRNA. In fact, RNA blotting analysis revealed that the mRNA is approximately 4Kb in size (see below). Subsequently, we screened other YT cDNA libraries by using the cloned cDNAs as probes, and several independent cDNA clones which together cover the entire mRNA for the IL-2R$\beta$ chain were isolated. Thus pIL-2R$\beta$6 and pIL-2R$\beta$19 were obtained by screening the cDNA libraries with the pIL-2R$\beta$9 cDNA insert in the probe.

The above mentioned plasmids containing cDNA coding for human IL-2R$\beta$ sequences have been deposited in strain E.coli MC 1061/P3 on March 2, 1989 at the Fermentation Research Institute according to the Budapest Treaty under the following accession numbers:

| Plasmid | Accession No. |
|---|---|
| pIL-2R$\beta$6 | FERM BP-2312 |
| pIL-2R$\beta$9 | FERM BP-2313 |
| pIL-2R$\beta$19 | FERM BP-2314 |
| pIL-2R$\beta$30 | FERM BP-2315 |

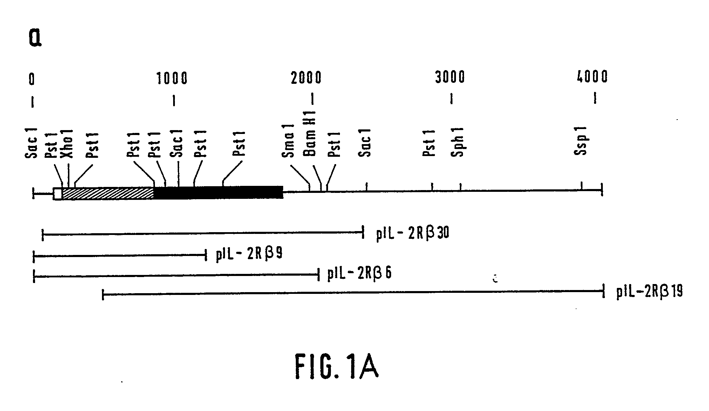

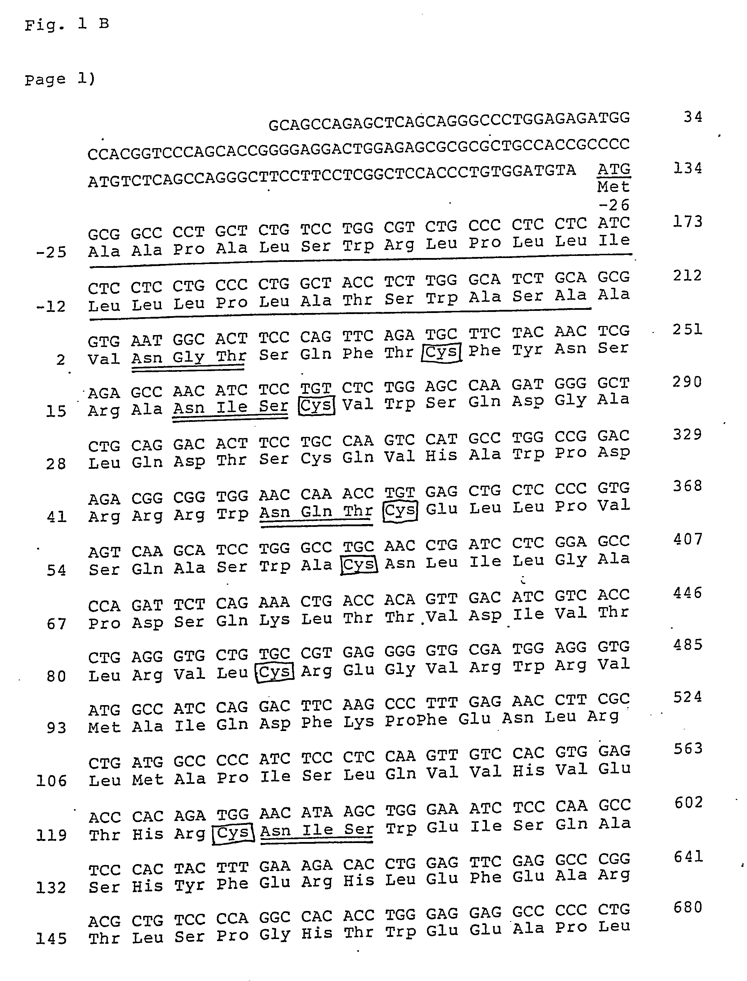

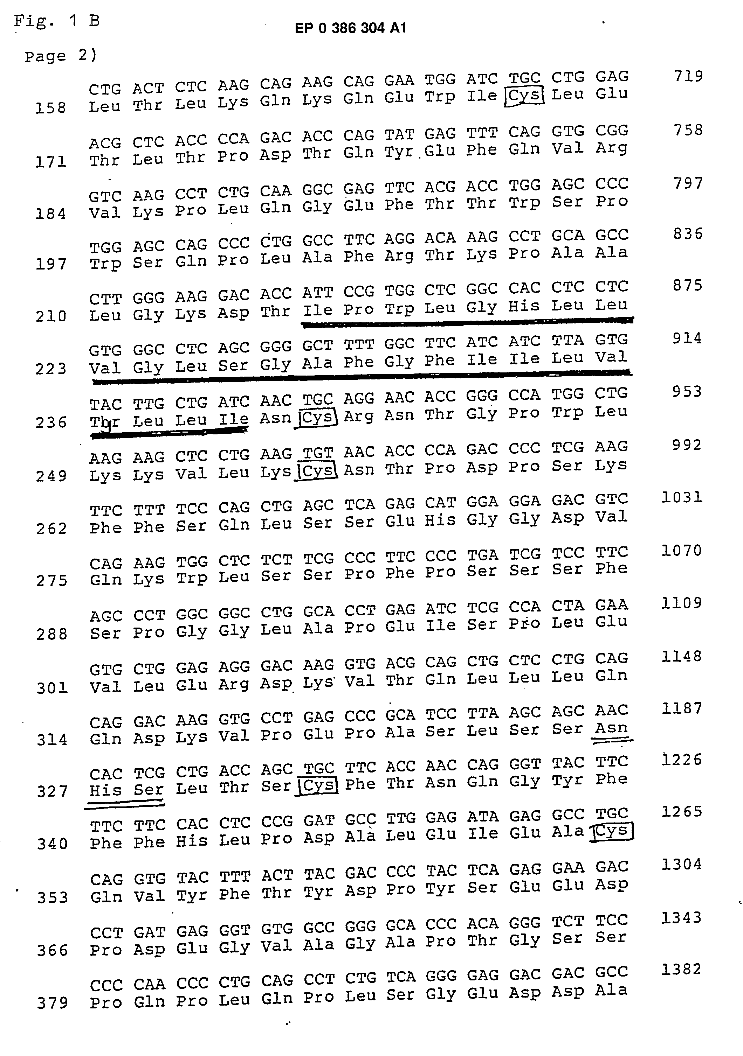

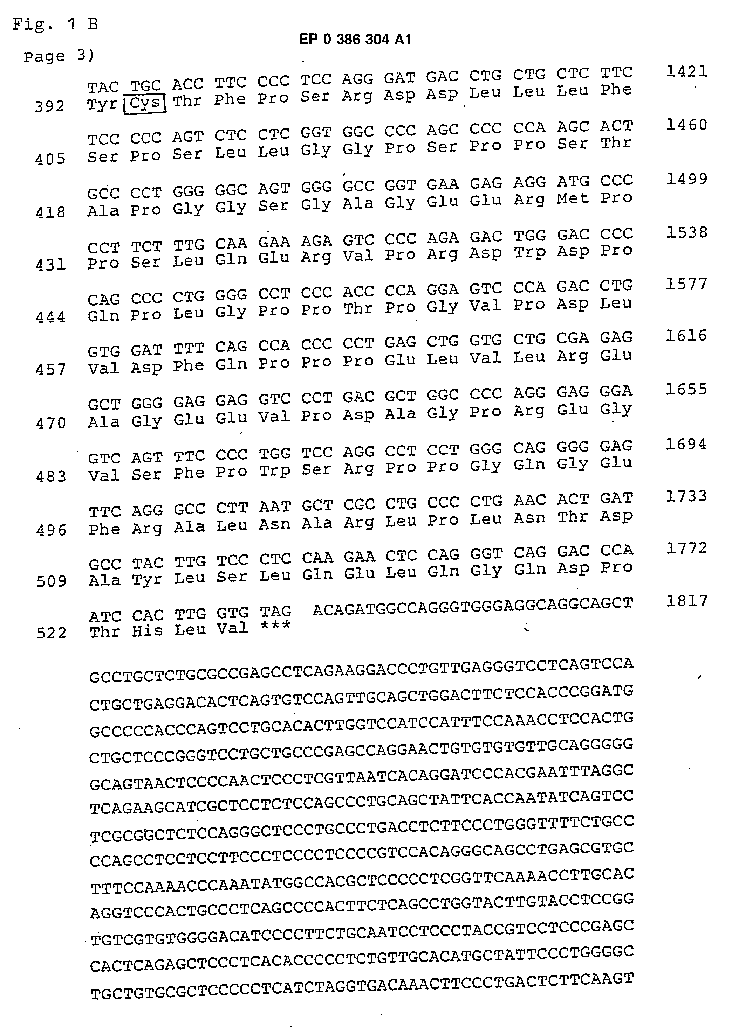

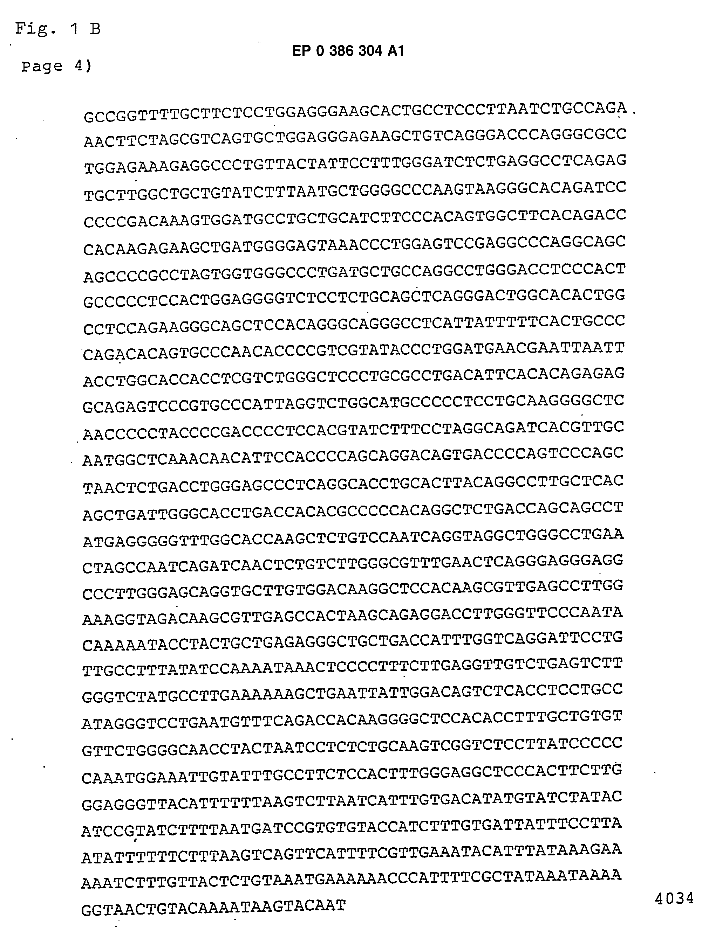

The complete nucleotide sequences of four of the cloned cDNAs were determined (Fig. 1).

Fig. 1 shows the structure of the human IL-2R$\beta$ chain cDNA. Fig. 1a is a schematic representation of the mRNA as deduced from the cloned cDNAs. Dotted, hatched, open and closed rectangles represent respectively the signal sequence, the extracellular, the transmembrane and the cytoplasmic regions of the mRNA are shown below. Fig. 1b shows the nucleotide and amino acid sequences of the human IL-2R$\beta$ chain cDNA. The sequence was deduced following the complete DNA sequence analysis of the above described cDNA clones. Nucleotides are numbered on the right margin and amino acids are numbered on the left margin. Clones pIL-2R$\beta$19 and pIL-2R$\beta$6 each contained G-A mutation at nucleotide residues 425 and 1531, respectively. Thus pIL-2R$\beta$6 cDNA acquired a TAG triplet in the cytoplasmic region. It is thought to be an error in reverse transcription, since all other clones, pIL-2R$\beta$30, pIL-2R8$\underline{1}$9 and pIL-2R$\beta$16 (28), have TGG triplet at that position. The first underlined 26 amino acid residues represent the signal sequence as predicted by the consensus sequence (22). The 25 transmembrane amino acid residues are marked with a thick underlining. The cysteine residues are boxed. The potential N-glycosylation sites are underlined twice. The possible poly-adenylation signals are shown by open rectangle. RNA was prepared from the NK-like human lymphoid cell line, YT, and cDNA libraries were prepared with CDM8 vector according to published procedures (21), except using either random primers (Amersham) (for pIL-2R$\beta$6, 9 and 30), or oligo (dT) primer (for pIL-2R$\beta$19). Screening of the cDNA libraries by a cocktail of anti-IL-2R$\beta$ monoclonal antibpdies, Mik-$\beta$1 and Mik-$\beta$2, was carried out as described previously (21). Nucleotide sequences were determined by a combination of dideoxy chain termination and chemical cleavage methods.

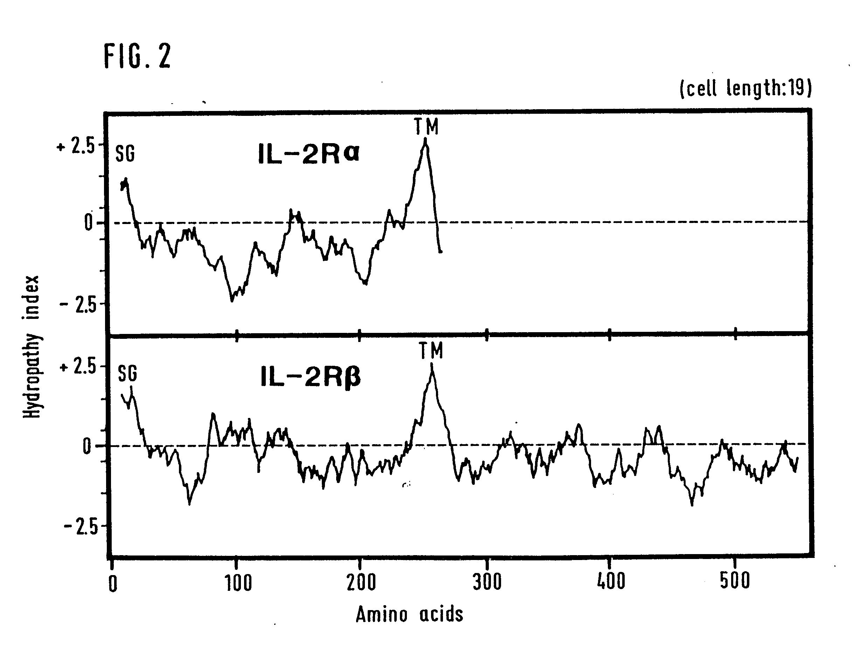

As shown in Fig. 1, the cDNA contains a large open reading frame that encodes a protein consisting of 551 amino acids. No significant homology with other known proteins was found in the Protein Sequence Database (National Biomedical Research Foundation, Washington, D.C.) or with sequences published more recently. Unlike many of other cytokine receptors, it appears that IL-2Rα and II-2Rβ chains do not belong to the immunoglobulin superfamily. From the deduced structure of the protein, the N-terminal 26 amino acids is considered to represent the signal sequence (Fig. 1 and 2) 22). Thus the mature form of the IL-2Rβ chain consists of 525 amino acids with a calculated M.W. of 58.358. As shown in Fig. 1, the receptor molecule consists of an extracellular region consisting of 214 amino acids. This region contains 8 cysteine residues of which 5 residues are found in the N-terminal half and they are interspaced rather periodically by 9-12 amino acids. It is likely that disulfide linkages between the cysteine residues impart a stable configuration for ligand binding. In fact, abundance of cysteine residues seems to be one of the common features of the ligand binding domain of many receptors (23). It may be worth noting that the predicted number of amino acids (a.a.) within the extracellular region of the II-2Rβ chain (214 a.a.) is almost comparable in number to that of the IL-2Rα chain (219 a.a.). Such size similarity may be significant in considering the conformation of the heterodimeric receptor complex that is quite unique for this receptor; as both α and β chains individually interact with distinct sites of the same IL-2 molecule (24).

A hydrophobic stretch of 25 amino acids spanning from the 215 to 239 amino acid residues appears to constitute the membrane spanning region of the receptor (Fig. 1 and 2).

Fig. 2 is a hydropathy plot analysis of deduced human IL-2Rα and II-2Rβ chain precursor structures. The analysis was carried out according to Kyte and Doolittle (38). SG and TM each represents signal sequence and transmembrane sequence, respectively.

The cytoplasmic region of the β chain consists of 286 a.a. and it is far larger than that of the α chain, which is only 13 a.a. long. The consensus sequences of tyrosine kinase (Gly-x-Gly-x-x-Gly) (25) are absent in the β chain. However, the presence of a triplet, Ala-Pro-Glu (293-295) may be noted; this has been asserted to be the consensus motif for a catalytic domain of some protein kinases (25). The possibility of the cytoplasmic region of the β chain having a protein kinase activity has yet to be tested. The primary structure of this region revealed yet another interesting feature; a rather strong bias for certain characteristic amino acids. This region is rich in proline (42/286) and serine (30/286) residues. Interestingly, the "proline rich" structure has also been demonstrated in the cytoplasmic region of CD2, a T cell membrance antigen involved in the activation pathway of T cells (26). The proline-rich structure may impart a non-globular conformation to this region that may be important in coupling of the receptor molecule with other signal transducer(s). The predominant serine residues may be the major target for phosphorylation, which could also modulate the receptor function (27). In addition, the cytoplasmic region is notably biased for negatively charged amino acids. In fact, this region contains 40 such amino acids (i.e. glutamic and aspartic acids), whereas only 18 amino acids account for the positively charged residues (i.e. lysine and arginine). Such a bias is particularly notable in the middle portion (a.a. 345-390) of the cytoplasmic region. Thus, the cytoplasmic region of the β chain may be quite acidic. Taken together some if not all of these unique characteristics may be responsible in driving further the downstream signal transduction pathway(s). The receptor protein contains 5 potential sites for N-linked glycosylation (Fig. 1), in which 4 are located in the extracellular region. Such a posttranslational modification may account for the difference between the M.W. of the estimated mature (70-75kD) and the calculated (58kD) protein molecules. Hydropathy plot analysis of the α and β chains revealed the presence of hydrophilic regions just adjacent to the cell membrane in the both chains (Fig. 2) These regions may play a role in the non-covalent intramolecular association between the two chains.

According to a one aspect of the present invention therefore we provide a recombinant cDNA coding for an IL-2Rβ chain or a portion thereof.

Preferably an cDNA of the invention has the formula:

GCAGCCAGAGCTCAGCAGGGCCCTGGAGAGATGG

CCACGGTCCCAGCACCGGGGAGGACTGGAGAGCGCGCGCTGCCACCGCCCC

ATGTCTCAGCCAGGGCTTCCTTCCTCGGCTCCACCCTGTGGATGTA    ATG

GCG GCC CCT GCT CTG TCC TGG CGT CTG CCC CTC CTC ATC

CTC CTC CTG CCC CTG GCT ACC TCT TGG GCA TCT GCA GCG

GTG AAT GGC ACT TCC CAG TTC AGA TGC TTC TAC AAC TCG

AGA GCC AAC ATC TCC TGT CTC TGG AGC CAA GAT GGG GCT

CTG CAG GAC ACT TCC TGC CAA GTC CAT GCC TGG CCG GAC

AGA CGG CGG TGG AAC CAA ACC TGT GAG CTG CTC CCC GTG

AGT CAA GCA TCC TGG GCC TGC AAC CTG ATC CTC GGA GCC

CCA GAT TCT CAG AAA CTG ACC ACA GTT GAC ATC GTC ACC

CTG AGG GTG CTG TGC CGT GAG GGG GTG CGA TGG AGG GTG

ATG GCC ATC CAG GAC TTC AAG CCC TTT GAG AAC CTT CGC

CTG ATG GCC CCC ATC TCC CTC CAA GTT GTC CAC GTG GAG

ACC CAC AGA TGG AAC ATA AGC TGG GAA ATC TCC CAA GCC

TCC CAC TAC TTT GAA AGA CAC CTG GAG TTC GAG GCC CGG

ACG CTG TCC CCA GGC CAC ACC TGG GAG GAG GCC CCC CTG

CTG ACT CTC AAG CAG AAG CAG GAA TGG ATC TGC CTG GAG

ACG CTC ACC CCA GAC ACC CAG TAT GAG TTT CAG GTG CGG

GTC AAG CCT CTG CAA GGC GAG TTC ACG ACC TGG AGC CCC

5

EP 0 386 304 A1

TGG AGC CAG CCC CTG GCC TTC AGG ACA AAG CCT GCA GCC
CTT GGG AAG GAC ACC ATT CCG TGG CTC GGC CAC CTC CTC
GTG GGC CTC AGC GGG GCT TTT GGC TTC ATC ATC TTA GTG
TAC TTG CTG ATC AAC TGC AGG AAC ACC GGG CCA TGG CTG
AAG AAG CTC CTG AAG TGT AAC ACC CCA GAC CCC TCG AAG
TTC TTT TCC CAG CTG AGC TCA GAG CAT GGA GGA GAC GTC
CAG AAG TGG CTC TCT TCG CCC TTC CCC TGA TCG TCC TTC
AGC CCT GGC GGC CTG GCA CCT GAG ATC TCG CCA CTA GAA
GTG CTG GAG AGG GAC AAG GTG ACG CAG CTG CTC CTG CAG
CAG GAC AAG GTG CCT GAG CCC GCA TCC TTA AGC AGC AAC
CAC TCG CTG ACC AGC TGC TTC ACC AAC CAG GGT TAC TTC
TTC TTC CAC CTC CCG GAT GCC TTG GAG ATA GAG GCC TGC
CAG GTG TAC TTT ACT TAC GAC CCC TAC TCA GAG GAA GAC
CCT GAT GAG GGT GTG GCC GGG GCA CCC ACA GGG TCT TCC
CCC CAA CCC CTG CAG CCT CTG TCA GGG GAG GAC GAC GCC
TAC TGC ACC TTC CCC TCC AGG GAT GAC CTG CTG CTC TTC
TCC CCC AGT CTC CTC GGT GGC CCC AGC CCC CCA AGC ACT
GCC CCT GGG GGC AGT GGG GCC GGT GAA GAG AGG ATG CCC
CCT TCT TTG CAA GAA AGA GTC CCC AGA GAC TGG GAC CCC
CAG CCC CTG GGG CCT CCC ACC CCA GGA GTC CCA GAC CTG
GTG GAT TTT CAG CCA CCC CCT GAG CTG GTG CTG CGA GAG
GCT GGG GAG GAG GTC CCT GAC GCT GGC CCC AGG GAG GGA
GTC AGT TTC CCC TGG TCC AGG CCT CCT GGG CAG GGG GAG
TTC AGG GCC CTT AAT GCT CGC CTG CCC CTG AAC ACT GAT
GCC TAC TTG TCC CTC CAA GAA CTC CAG GGT CAG GAC CCA
ATC CAC TTG GTG TAG   ACAGATGGCCAGGGTGGGAGGCAGGCAGCT
GCCTGCTCTGCGCCGAGCCTCAGAAGGACCCTGTTGAGGGTCCTCAGTCCA
CTGCTGAGGACACTCAGTGTCCAGTTGCAGCTGGACTTCTCCACCCGGATG
GCCCCCACCCAGTCCTGCACACTTGGTCCATCCATTTCCAAACCTCCACTG
CTGCTCCCGGGTCCTGCTGCCCGAGCCAGGAACTGTGTGTGTTGCAGGGGG
GCAGTAACTCCCCAACTCCCTCGTTAATCACAGGATCCCACGAATTTAGGC
TCAGAAGCATCGCTCCTCTCCAGCCCTGCAGCTATTCACCAATATCAGTCC
TCGCGGCTCTCCAGGGCTCCCTGCCCTGACCTCTTCCCTGGGTTTTCTGCC
CCAGCCTCCTCCTTCCCTCCCCTCCCCGTCCACAGGGCAGCCTGAGCGTGC
TTTCCAAAACCCAAATATGGCCACGCTCCCCCTCGGTTCAAAACCTTGCAC

6

AGGTCCCACTGCCCTCAGCCCCACTTCTCAGCCTGGTACTTGTACCTCCGG
TGTCGTGTGGGGACATCCCCTTCTGCAATCCTCCCTACCGTCCTCCCGAGC
CACTCAGAGCTCCCTCACACCCCTCTGTTGCACATGCTATTCCCTGGGGC
TGCTGTGCGCTCCCCCTCATCTAGGTGACAAACTTCCCTGACTCTTCAAGT
GCCGGTTTTGCTTCTCCTGGAGGGAAGCACTGCCTCCCTTAATCTGCCAGA
AACTTCTAGCGTCAGTGCTGGAGGGAGAAGCTGTCAGGGACCCAGGGCGCC
TGGAGAAAGAGGCCCTGTTACTATTCCTTTGGGATCTCTGAGGCCTCAGAG
TGCTTGGCTGCTGTATCTTTAATGCTGGGGCCCAAGTAAGGGCACAGATCC
CCCCGACAAAGTGGATGCCTGCTGCATCTTCCCACAGTGGCTTCACAGACC
CACAAGAGAAGCTGATGGGGAGTAAACCCTGGAGTCCGAGGCCCAGGCAGC
AGCCCCGCCTAGTGGTGGGCCCTGATGCTGCCAGGCCTGGGACCTCCCACT
GCCCCCTCCACTGGAGGGGTCTCCTCTGCAGCTCAGGGACTGGCACACTGG
CCTCCAGAAGGGCAGCTCCACAGGGCAGGGCCTCATTATTTTTCACTGCCC
CAGACACAGTGCCCAACACCCCGTCGTATACCCTGGATGAACGAATTAATT
ACCTGGCACCACCTCGTCTGGGCTCCCTGCGCCTGACATTCACACAGAGAG
GCAGAGTCCCGTGCCCATTAGGTCTGGCATGCCCCCTCCTGCAAGGGGCTC
AACCCCCTACCCCGACCCCTCCACGTATCTTTCCTAGGCAGATCACGTTGC
AATGGCTCAAACAACATTCCACCCCAGCAGGACAGTGACCCCAGTCCCAGC
TAACTCTGACCTGGGAGCCCTCAGGCACCTGCACTTACAGGCCTTGCTCAC
AGCTGATTGGGCACCTGACCACACGCCCCCACAGGCTCTGACCAGCAGCCT
ATGAGGGGGTTTGGCACCAAGCTCTGTCCAATCAGGTAGGCTGGGCCTGAA
CTAGCCAATCAGATCAACTCTGTCTTGGGCGTTTGAACTCAGGGAGGGAGG
CCCTTGGGAGCAGGTGCTTGTGGACAAGGCTCCACAAGCGTTGAGCCTTGG
AAAGGTAGACAAGCGTTGAGCCACTAAGCAGAGGACCTTGGGTTCCCAATA
CAAAAATACCTACTGCTGAGAGGGCTGCTGACCATTTGGTCAGGATTCCTG
TTGCCTTTATATCCAAAATAAACTCCCCTTTCTTGAGGTTGTCTGAGTCTT
GGGTCTATGCCTTGAAAAAAGCTGAATTATTGGACAGTCTCACCTCCTGCC
ATAGGGTCCTGAATGTTTCAGACCACAAGGGGCTCCACACCTTTGCTGTGT
GTTCTGGGGCAACCTACTAATCCTCTCTGCAAGTCGGTCTCCTTATCCCCC
CAAATGGAAATTGTATTTGCCTTCTCCACTTTGGGAGGCTCCCACTTCTTG
GGAGGGTTACATTTTTTAAGTCTTAATCATTTGTGACATATGTATCTATAC
ATCCGTATCTTTTAATGATCCGTGTGTACCATCTTTGTGATTATTTCCTTA
ATATTTTTTCTTTAAGTCAGTTCATTTTCGTTGAAATACATTTATAAAGAA
AAATCTTTGTTACTCTGTAAATGAAAAAACCCATTTTCGCTATAAATAAAA
GGTAACTGTACAAAATAAGTACAAT

which codes for human II-2R$\beta$ or in a degenerate variant thereof or a portion thereof.

The present invention thus also includes cDNA coding for portions of the complete sequence of the human IL-2R$\beta$ chain for instance the extracellular portion beginning at, or about amino acid (a,a) (see Fig. 1 B) 1 e.g. 1, 2, 3, 4, 5, 6, 7, 8, 9, 10 and ending at or about a.a. 214 e.g. 200, 201, 202, 203, 204, 205, 206, 207, 208, 209, 210, 211, 212, 214, 215, 216, 217, 218, 219, 220, or sub-portions of this extracellular part, or portions corresponding to the intracellular part of the receptor chain e.g. the portion beginning at or about a.a. 239 e.g. a.a. 230, 231, 232, 234, 235, 236, 237, 238, 239, 240, 241, 242, up to or about the end a.a. 525, e.g. 516, 517, 518, 519, 520, 521, 522, 523, 524 and 535.

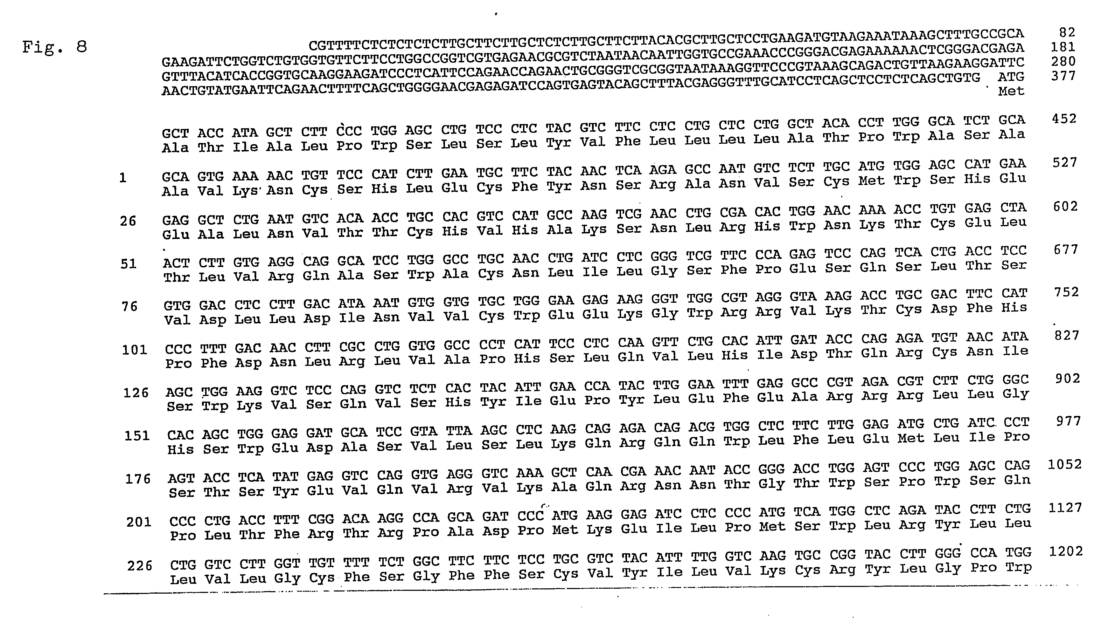

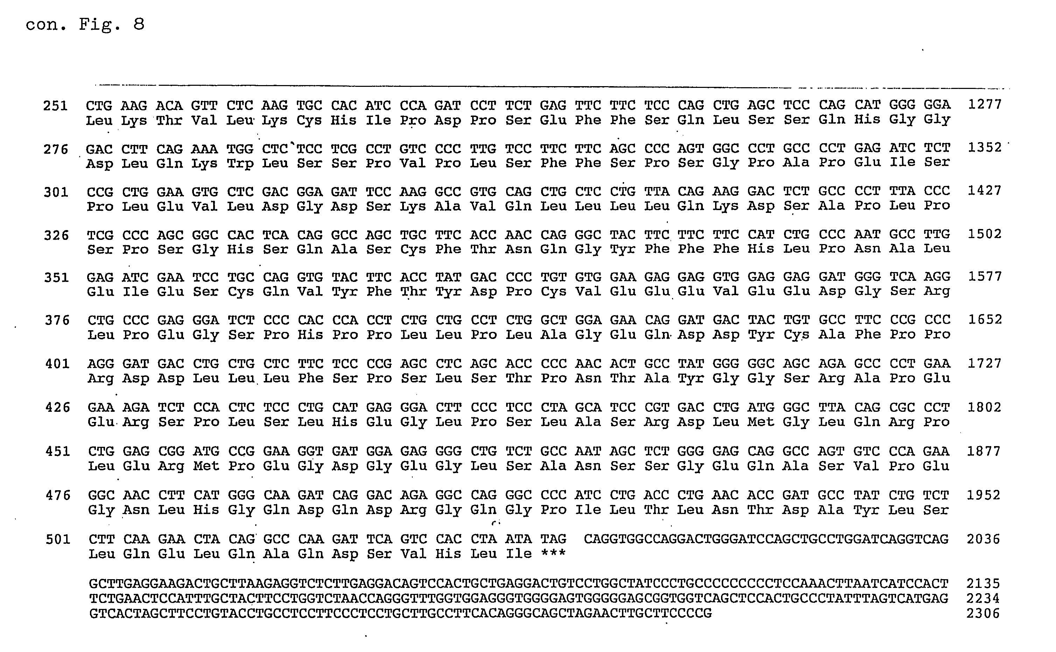

The present invention also includes a cDNA coding for murine IL-2R$\beta$ chain i.e. the cDNA sequence coding only for amino acids to 513 in Fig. 8 or a degenerate variant therof as well as to the entire neucleotide sequence as set forth in Fig. 8 or a degenerate variant therof, and to cDNA coding for portions of the said sequence, for instance the extra-cellular part beginnning at, or about amino acid 1 e. g. 1, 2, 3, 4, 5, 6, 7, 8, 9, 10 and ending at or about amino acid 210 e.g. 200, 201, 202, 203, 204, 205, 206, 207, 208, 209, 210, 211,212, 213, 214, 215, 216, 217, 218, 219, 210 or sub-portions of their extracellular part, or portions corresponding to the intracellular part of the receptor chain e.g. the portion beginning at or about amino acid 235 e.g. amino acid 225, 226, 227, 228, 229, 230, 231, 232, 233, 234, 235, 236, 237, 238, 239, 240, 241, 242, up to or about the end a.a. 513 e.g. 505, 506, 507, 508, 509, 510, 512, and 513.

The present invention also includes cDNA sequences capable of hybridising with the DNA sequences as described herein and having the activity of an IL-2R$\beta$ chain.

Using standard techniques of recombinant DNA technology vectors for transforming suitable host cells can be constructed which contain cDNA sequences corrsponding to the structural gene for IL-2R$\beta$ as set forth above or any derived portion thereof, or a degenerate variant thereof.

Suitable vectors are plasmid vectors for example and will include control and regulatory sequences operably linked to the cDNA sequence coding for the IL-2R$\beta$ chain or portion thereof.

Suitable techniques are well known and widely practised and by way of Example are described, in connection with other proteins in European Patent Applications, Publication Nos. 0254249 and 0170204.

Obtaining the desired portion in pure form from the culture can be carried out by standard techniques and such protein provides a suitable antigen for preparing monoclonal antibodies. Thus hybridomas capable of secreting a monoclonal antibody having a specific affinity to the IL-2R$\beta$ chain or a desired portion thereof may be prepared by immunizing a non-human animal with recombinant IL-2R$\beta$ or a portion thereof, removing spleen cells with non-immunoglobulin secreting myclonas cells, and selecting from the resulting hybridomas a cell line which produces a monoclonal antibody having the desired binding specificity and, if desired, subsequently sub-cloning said hybridoma.

The techniques for preparing hybridomas and obtaining monoclonal antibodies in pure form therefrom are well known and by way of example are described in European Patent Application, Publication No. 0168745.

Antibodies in accordance with the invention are useful e.g. for diagnostic purposes and also for therapy by immune suppression. or activation. As mentioned above, such antibodies could be raised using purified recombinant protein in accordance with the invention or by transfecting the cDNA of the invention, obtaining cells expressing large amounts of the receptor and using such cells to obtain the antibodies.

The present invention envisages soluble forms of IL-2R$\beta$ chain and of soluble IL-2 receptor. That is the IL-2R8 chain may be produced in soluble form or the $\alpha$-chain and $\beta$-chain produced simultaneously.

The availability of monoclonal antibodies to specific sub-portions of the IL-2$\beta$ chain enables epitopes of the receptor chain to be identified and thus opens the way for control of the activity of the receptor to be excerised using suitable monoclonal antibodies or other peptides or peptide mimetic or protein analogues substances.

## Expression of Human IL-2R$\beta$ chain mRNA

Expression of the IL-2R$\beta$ mRNA was examined by using the cDNA insert from pIL-2R$\beta$30 as the probe

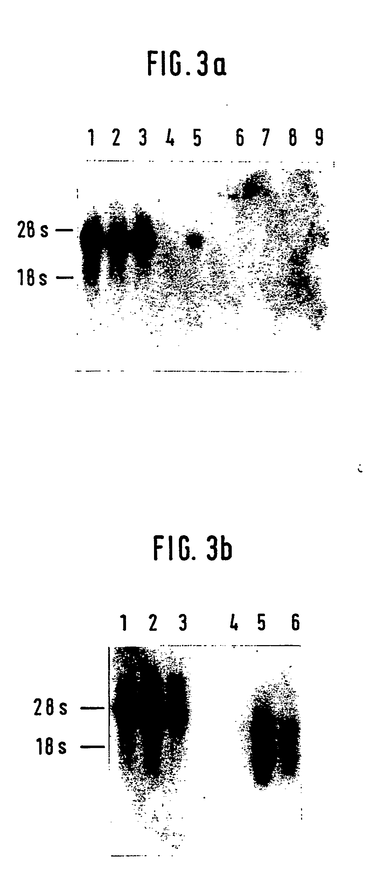

Fig. 3a illustrates the expression of human IL-2R$\beta$ chain mRNA in different cell types. Poly(A)$^+$RNA (2$\mu$g per lane) from the following cell sources was prepared and subjected to RNA blotting analysis using the XhoI-digested human IL-2R$\beta$ chain cDNA fragment derived from pIL-2R$\beta$30 as a probe following standard procedures (14, 18, 27). Lane 1, YT; lane 2, Hut102(HTLV-1 transformed human T cell line); lane 3, MT-2(HTLV-1 transformed human T cell line); lane 4, ARH-77 (multible myeloma line); lane 5, SKW6.4 (EBV-tranformed human $\beta$ lymphoblastoid line); lane 6, U937 (histiocytic leukemia line); lane 7, MT-1 (HTLV-1 transformed human T cell line); lane 8, Jurkat (human T leukemic line); lane 9, HeLa (human cervical carcinoma cell line.

As shown in Figure 3a, the RNA blot analysis revealed the presence of a 4kb mRNA, the expression of which is restricted to lymphoid cells previously identified to bear IL-2R$\beta$ chain (i.e. YT, MT-2, Hut102, SKW6.4) (12, 16, 17). On the other hand, the mRNA expression was not detected in cells such as Jurkat, MT-1, U937, ARH-77 and HeLa cells. Essentially, the mRNA expression levels are in correlation with the IL-2R$\beta$ chain expression levels.

Fig. 3 b illustrates the expression of IL-2R$\beta$ and Il-2R$\alpha$ mRNAS in human PBLs. Total RNA (15$\mu$g per lane) was loaded in each lane. Lanes 1 and 4 represents unstimulated human peripheral blood lymphocytes (PBLs); lanes 2 and 5, PBLs stimulated with 5$\mu$g/ml phytohemagglutinin (PHA) for 24 hrs; lanes 3 and 6, PBLs stimulated with 5$\mu$g/ml PHA for 72 hrs. The RNA-blotted filter was hybridized with the IL-2R$\beta$ probe (lanes 1-3). After dehybridization of the IL-2R$\beta$ probe, the same filter was hybridized with the IL-2R$\alpha$ probe (Xbal-Bcll fragment derived from pSVIL2R-3 (14) (lanes 4-6).

Interestingly, the IL-2R$\beta$ mRNA was detectable in the unstimulated PBLs and its expression levels increased transiently only 2.5-fold after mitogen stimulation. Based on previous data derived from flow cytometric analysis (19), it is likely that the mRNA induction patterns differ between the different lymphocyte populations. This expression pattern is quite different from that of the IL-2R$\alpha$ chain whose expression strictly requires mitogenic stimulation of the cells (Fig. 3b), suggesting the presence of distinct mechanisms of gene expression between the two genes.

Southern blot analysis of the genomic DNA from PBL and various cell lines including HTLV-1-transformed human T cell lines indicates that the gene is present in a single copy and is not rearranged in those cells.

## IL-2 binding properties of the cDNA-encoded IL-2R$\beta$ chain

We next carried out a series of cDNA expression studies in order to examine if the cDNA product binds IL-2 and indeed manifests the properties of the IL-2R$\beta$ chain that have been demonstrated and/or suggested in previous studies. Two cDNA expression plasmids were constructed in which expression of the cDNA spanning the entire coding region was directed by either the mouse lck gene (29) promoter (pLCKR$\beta$) or Moloney leukemia virus LTR (30) (pMLVR$\beta$).

Expression vectors were constructed by the following procedures. pIL-2R$\beta$30 was digested with HindIII (the cleavage site is located within the polylinker regions of CDM8) and, after fill-in of both ends, a BamHI linker was attached and religated. The resulting plasmid was then digested with BamHI and the 1.8kb DNA fragment which contains the entire coding sequence for the $\beta$ chain was introduced into BamHI-cleaved p1013 vector containing the mouse lck promoter to construct pLCKR$\beta$. The BamHI-digested cDNA fragment was also introduced into a retrovirus vector, pZipSV(X) (30), to construct pMLVR$\beta$. The human IL-2R$\alpha$ expressing vector, pSVIL2Rneo, was obtained from pSVIL2R-3 (14) by replacing the Eco-gypt gene with the neo-resistance gene.

The plasmid pLCKR$\beta$ was introduced into the mouse T lymphoma EL-4 and the human T cell leukemia Jurkat lines, both of which are known to be devoid of surface molecules that bind human IL-2.

Transfection of the expression plasmids into Jurkat and EL-4 cells was carried out by electroporation as described previously (39). Transfected cells were selected in the RPMI1640 medium containing 10% fetal calf serum (FCS) and G418 (1 mg/ml for EL-4 and 1.5 mg/ml for Jurkat). To obtain cells expressing cDNAs for human IL-2R$\alpha$ and IL-2R$\beta$ chains simultaneously, a Jurkat-derived clone J$\alpha$-5, transfected with pSVIL2Rneo, was co-transfected with pLCKR$\beta$ and a plasmid containing the hygromycin-resistance gene, pHgy. The transfected cells were selected with 200pg/ml hygromycin. Transfection of pMLVR$\beta$ into 2 cells was carried out by calcium-phosphate method as described previously (14) and the cells were selected by 700$\mu$g/ml of G418. For flow cytometric analysis, 5x10$^5$ cells were treated with antibody (1:500 dilution of ascites) at 4°C for 30 min. After washing, cells were stained with fluorescein-conjugated goat anti-mouse IgG.

The stained cells were analysed on a FACS440 flow cytometer (Beckton Dickinson). The $^{125}$I-IL-2 binding assay and Scatchard plot analysis were carried out as described previously (12).

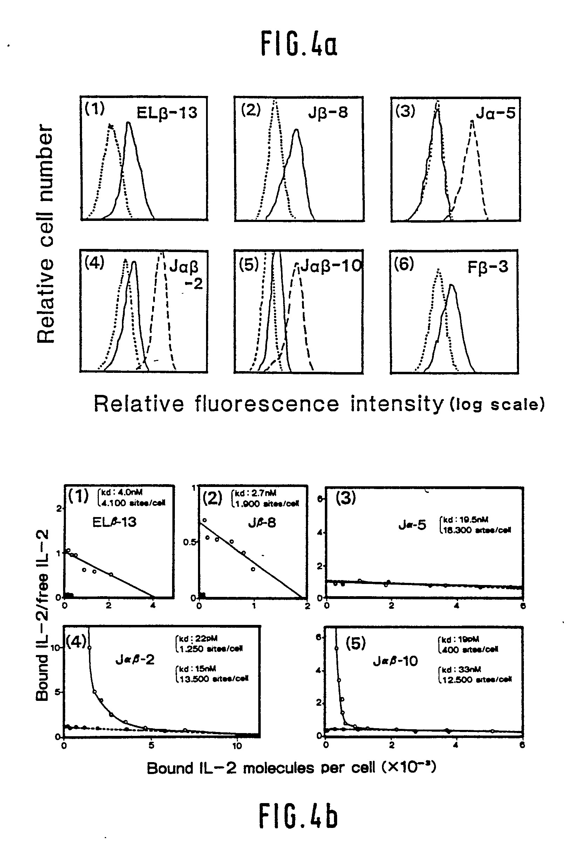

Fig. 4a illustrates the expression of human IL-2R$\alpha$ and/or IL-2R$\beta$ chain cDNAs by means of cell surface staining patterns of human IL-2R$\alpha$ and/or IL-2R$\beta$ cDNA transfectants. Parental cells and various transfectant cells were separately stained with either a monoclonal anti-human IL-2R$\alpha$ antibody, anti-Tac (-----), or monoclonal anti-human IL-2R$\beta$ antibody, Mik-$\beta$1 (————), Dotted line (.....) is a fluorescence profile of the cells stained with fluorescein-conjgated goat-anti-mouse IgG alone. Cells used were (1) EL$\beta$-13 (and EL-4-derived clone transfected with pLCKR$\beta$), (2) J$\beta$-8 (a Jurkat-derived clone transfected with pLCKR$\beta$), (3)

9

Jα-5 (a Jurkat-derived clone transfected with pSVIL2Rneo), (4) Jα-2 (a Jα-5-derived clone transfected with pLCKRβ), (5) Jαβ-10 (a Jα-5-derived clone transfected with pLCKRβ), and (6) FB-3 (a NIH3T3-derived line transfected with pMLVRβ).

Stable transformant clones expressing the cDNA product were obtained for both the EL-4 (ELβ-13) and Jurkat (Jβ-8 and Jβ-9) cells as judged by FACS analysis (Fig. 4a). In addition, we also introduced the same gene into the Jurkat transformant clone, Jβ-5, which expresses the transfected, human IL-2Rα chain cDNA. Two of the resulting transformants, Jαβ-2 and Jαβ-10, were found to express both α and β chains (Fig. 4a-(4), (5)). As expected, RNA blotting analyses of the mRNA expressed in those transformants revealed that the α and β chain-specific mRNAS are derived from the transfected cDNAs but not from the endogenous genes (26). Furthermore, in order to examine the property of the cDNA product in non-lymphoid cells, the plasmid pMLVRβ was introduced into an NIH3T3 cell-derived cell line 2 (30), and the resulting transformant expressing the cDNA, Fβ-3, was obtained (Fig. 4a-(5)).

The IL-2 binding studies were carried out with $^{125}$I-labeled, recombinant human IL-2.

Fig. 4b illustrates the expression of the α and β chains by means of the Scatchard plot analysis of $^{125}$I-IL-2 binding to the transfectants expressing the cloned cDNAs. Scatchard plot of the IL-2 binding data in the absence ( - ) or presence ( - ) of 1:100-diluted ascites of Mik-β1. Binding of $^{125}$I-IL-2 to ELβ-13 or Jβ-8 was completely abolished by Mik-β1. No specific IL-2 binding was observed when parental Jurkat or EL-4 cells were examined. The number of IL-2 binding sites per cell and the receptor affinity were determined by computer-assisted analysis of the IL-2 binding data. (1) ELβ-13, (2) Jβ-8, (3) Jα-5, (4) Jαβ-2, (5) Jαβ-10.

As can be seen the EL-4-derived clone (ELβ-13) and the Jurkat-derived clone (Jβ-8), both expressing the β chain cDNA displayed intermediate-affinity to IL-2 with estimated Kd values of 4.0nm and 2.7nm, respectively. The IL-2 binding to those cells was completely abolished by the Mik-β1 antibody (Fig. 4b-(1), (2)). The Jurkat-derived Jαβ-2 and Jαβ-10 clones expressing both the human IL-2Rα and II-2Rβ cDNA displayed both high and low affinity receptors with estimated Kp values of 22pM and I5nm for Jαβ-2 and 19pM and 33nm for Jαβ-10, respectively. In contrast, the parental, Jurkat-derived Jα-5 cells expressing the α chain cDNA alone manifested exclusively low-affinity (Kd: 19.5nM) to IL-2 (Fig. 4b-(3)). the number of the high-affinity IL-2R expressed Jαβ-2 cells and Jαβ-10 was comparable to that of expressed IL-2Rβ molecules. In addition, treatment of these cells with Mik-β1 antibody completely abolished high-affinity IL-2 binding sites from the cell surface, while retaining the expression of low-affinity IL-2R (Fig. 4b-(4), (5)). These observations demonstrate unequivocally that the CDNA-encoded IL-2Rβ molecule is directly involved in the formation of high-affinity receptor complex in association with the IL-2Rα chain. In contrast to the aforedescribed T cell transformants, the Fβ-3 cells did not display any IL-2 binding on the cell surface under same binding conditions. Interestingly the same observation was made with monkey COS cells that express the β chain, but failed to bind IL-2 (28). Thus, the results suggest the involvement of either a cell-type specific processing mechanism(s) or an additional cellular component(s), or both for the functional IL-2Rβ chain expression.

In order to characterize further the molecular structure of reconstituted IL-2R, we performed chemical crosslinking experiments with $^{125}$I-IL-2 and non-cleavable chemical crosslinker, dissuccinimidyl suberate (DSS).

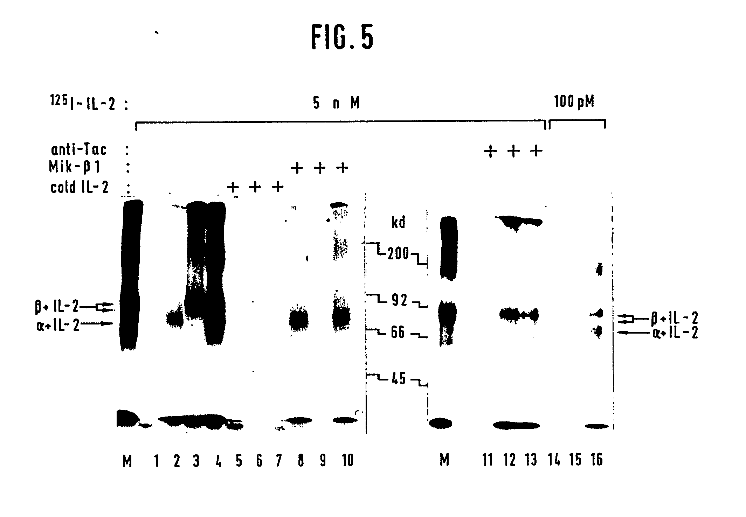

Fig. 5 illustrates the results of the affinity cross-linking studies of the IL-2R-positive transformants. Cells were incubated with 5nm (lanes 1-13) or 100pM (lanes 14-16) of $^{125}$I-IL-2 in the absence (lanes 1-4, 14-16) or presence of a 250-fold molar excess of unlabeled IL-2 (lanes 5-7), 500-fold molar excess of affinity column-purified Mik-β1 (lanes 8-10) or 500-fold molar excess of affinity column-purified anti-Tac (lanes 11-13). Then cells were chemically crosslinked with dissuccinimidyl suberate (DSS) as described previously (16). The cells were then solubilized and the supernatants were subjected to 7.5% SDS-PAGE. Cells used were: Jurkat (lane 1); Jα-5 (lanes 2, 5, 8, 11, 14); Jβ-8 lanes 3, 6, 9, 12, 15); Jαβ-10 (lanes 4, 7, 10, 13, 16). YT cells crosslinked with $^{125}$I-IL-2 were used as a marker (M).

As can be seen cells expressing only IL-2Rβ chain were crosslinked with $^{125}$I-labeled IL-2 and analysed by SDS-PAGE, a doublet band consisting of 90kD major and 85kD minor was detected and its migration profile was indistinguishable from that of YT cells (see arrows in Fig. 5 and ref. 16, 17). The appearance of the doublet is inhibited by an excess of unlabeled IL-2 or by Mik-β1. The doublet formation may be due to degradation of receptor-IL-2 complex. It is also possible that both protein products are derived by a differential post-translational modification(s). Alternatively, one of the doublet may represent a third component of the receptor complex. A broad band migrating around the position of 150kD was also detected in the transfectant (Jαβ-10) as well as YT cells. The appearance of this band is also inhibited by either unlabeled IL-2 or Mik-β1. It may represent the ternary complex of IL-2, IL-2Rα and II-2Rβ molecules. In a series of chemical cross-linking experiments shown in Fig. 4, it was demonstrated that the physico-chemical properties of the receptor complex expressed on the surface of Jαβ-2 are indistinguishable from the properties of high-affinity receptor expressed on cultured T cells or PBLs (12, 16, 17).

Preliminary results of experiments to determine whether the expression of the $\alpha$ and $\beta$ chains in non-lymphoid cells results in the formation of high-affinity receptor indicate that, when the $\alpha$ and $\beta$ chain cDNAS are co-expressed transiently in COS cells, both chains can crosslink with $^{125}$I-IL-2 at the concentration (400 pM) in which the similarly expressed $\alpha$ chain alone can not (28). The results may suggest the formation of the $\alpha\beta$ heterodimeric receptor in this non-lymphoid cell line.

IL-2 internalization by reconstituted receptors

It has been reported that intermediate- and high-affinity IL-2 receptors can both internalize IL-2 (33-35). Ligand internalization is usually accompanied with the IL-2 signal transduction, suggesting this process to be essential.

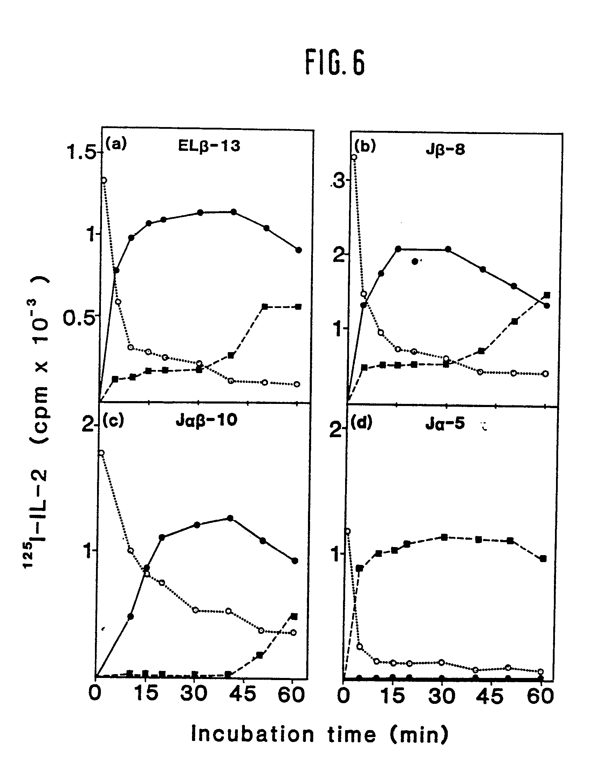

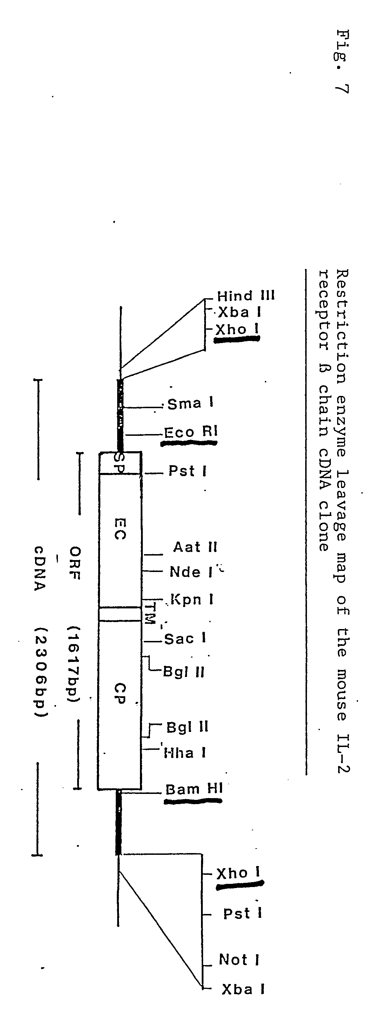

Fig. 6 illustrates IL-2 internalization via the reconstituted receptors. IL-2 internalization was examined according to a method described previously (33). Briefly, cells ($5\times10^7$) were treated with $^{125}$I-IL-2 at a final concentration of 200pM (J$\alpha\beta$-10) or 5nM (J$\alpha$-5, J$\beta$-8 and EL$\beta$-13) at 0°C for 30 min. After washing, cells were suspended with prewarmed culture medium (37°C) and the kinetics of IL-2 internalization was examined as described previously (33). (a) EL$\beta$-13, (b) J$\beta$-8, (c) J$\alpha\beta$-10, (d) J$\alpha$-5. (- - - -), internalized IL-2; (... ... ...), cell-surface bound IL-2; (- - - - -), free IL-2.

As shown in Fig. 6, we examined whether the reconstituted receptors can internalize IL-2. In fact, the cells expressing IL-2R$\beta$ chain alone, or both $\alpha$ and $\beta$ chains are capable of internalizing IL-2 following a kinetic pattern similar to that reported for the native receptor. In contrast, the Jurkat cells expressing only IL-2R$\alpha$ failed to internalize IL-2, similar to previously reported observations (33, 34). Preliminary results indicate that the growth of the cells expressing the intermediate- or high-affinity receptors is selectively inhibited by IL-2 (14, 36). We also have preliminary results that the $\beta$ chain expressed in another host cell line functions in stimulating the cell growth in response to IL-2 (28).

Cloning of Murine IL-2R receptor $\beta$ chain

No specific antibodies to murine IL-2 receptor $\beta$-chain are known to exist, accordingly the screening method used for the isolation of cNDA for Hu IL-2R$\beta$ chain was not employed.

A cDNA library was prepared using poly (A)$^+$-RNA from Concanavalin A stimulated mouse spleen cells; the cDNA was cloned in $\lambda$gt 10 which was multiplied in E. coli. Screening of this library was then carried out using the above described human IL-2R$\beta$ chain cDNA as the probe under non-stringent conditions. From the positive clones a clone designated $\lambda$MIL 2R$\beta$-26 was selected. The cDNA insert in this clone contained only a 540 bp sequence of the whole murine Il-2R$\beta$ chain sequence. This sequence was therefore isolated by digestion of $\lambda$MIL-2R$\beta$-26 using Pvu 2 and used for screening another cDNA library prepared using poly (A)$^+$ from the mouse thymoma cell line EL-4 according to standard procedures and cloned into the BstXI site of the CDM8 vector followed by transfecting E. coli.

Screening of the cDNA library was carried out under highly stringent conditions according to the method described in European Patent Application No. 88 119 602.9 and Kashima et al. (Nature Vo. 313 pp 402-404, 1985).

From the positive clones clone pMIL-2R$\beta$-36 containing the structural gene for murine IL-2R$\beta$ was selected.

Plasmid pMIL-2R$\beta$-36 has been deposited in strain E. coli MC 1061/P3 on May 23, 1989 at the Fermantation Research Institute according the Budapest Treaty under accession number FERM BP-2435.

The availability of the genes encoding IL-2R$\beta$ chains makes it possible to explore novel approaches for the functional studies of the IL-2 system. The receptor structure operating in the IL-2 system is unique in that two structurally distinct membrane molecules, the IL-2R$\alpha$ and IL-2R$\beta$ chains, both bind IL-2 independently. The series of cDNA expression examples described herein substantiate further the previous notion that the $\alpha$ and $\beta$ chains constitute the high-affinity IL-2R complex via a non-covalent association of the molecules (18, 37). Thus the peculiarity of this system is the involvement of three intermolecular interactions between one ligand and two distinct receptors. By virtue of the present invention it will now be possible to elucidate functional domains of this unique cytokine receptor system. Mutational analyses of the cloned $\beta$ chain cDNA may provide clues as to the identification of respective domains involved in ligand binding and association with the $\alpha$ chain. To date, little is known about the cascade of biochemical events triggered by cytokines interacting with their homologous receptors. By the present invention we have demonstrate the presence in the IL-2R$\beta$ chain of a large cytoplasmic region which most likely is involved in driving the IL-2 signal pathway(s). The particular acidic nuclei found in the cytoplasmic region may suggest coupling to other cytoplasmic signal transducers. Alternatively, in view of a previous report on the presence of IL-2 within the nucleous (33), an intriguing possibility is that the acidic as well as the proline-rich regions of the IL-2R$\beta$ cytoplasmic component may play a role in activation of the genetic programming. The availability of the expression system in which the cDNA-encoded $\beta$ chain can deliver growth signals will allow further clarification of the functional domaines of the receptor. It is now possible to study the essential role of IL-2 in the development and regulation of the immune system.

## REFERENCES AND NOTES

1. D.A. Morgan, F.W. Ruscelli, R.C. Gallo, Science 198, 1007 (1976)

2. K.A. Smith, Annu.Rev.Immunol. 2, 319 (1984); T. Taniguchi et al., Immunol.Rev. 92, 121 (1986)

3. D.H. Raulet, Nature 314, 101 (1985)

4. M. Tsudo, T. Uchiyama, H. Uchino, J.Exp.Med. 160, 612 (1984); T.A. Waldmann et al., ibid., p. 1450 (1984); M.A. Blackman, M.A. Tigges, M.E. Minis, M.E. Koshland, Cell, 47, 609 (1986)

5. M. Malkovsky et al., Nature 325, 262 (1987)

6. C.S. Henney, K. Kuribayashi, D.E. Kern, S. Gillis, ibid. 291, 335 (1981)

7. M.E. Lotze, E.A. Grimm, S.A. Strausser, S.A. Rosenberg, Cancer Res. 41, 4420 (1981); E.A. Grimm, A. Mazumder, H.Z. Zhang, S.A. Rosenberg, J.Exp. Med. 155, 1823 (1982

8. S.A. Rosenberg et al., N.Engl.J.Med. 316, 889 (1987)

9. E.N. Benveniste, J.E. Merrill, Nature 321, 610 (1986)

10. S.J. LeGrue, Lymphokine Res. 7, 1987 (1988); G.B. Millis, P. Girard, S. Grinstein, E.W. Gelfand, Cell 55, 91 (1988); V.E. Valge, J.G.P. Wong, B.M. Datlof, A.J. Sinskey, A. Rao, ibid., p. 101 (1988); M.A. Tigges, L.S. Casey, M.E. Koshland, Science 243, 781 (1989)

11. R.J. Robb, W.C. Greene, C.M. Rusk, J.Exp.Med. 160, 1126 (1984)

12. M. Tsudo, R.W. Kozak, C.K. Goldman, T.A. Waldmann, Proc.Natl.Acad.Sci.U.S.A. 83, 9694 (1986); K. Teshigawara, H.-M. Wang, K.Kato, K.A. Smith, J.Exp.Med. 165, 223 (1987)

13. W.J. Leonard et al., Nature 311, 626 (1984) T. Nikaido et al., ibid., p. 631 (1984); D. Cosman et al., ibid. 312, 768 (1984)

14. M. Hatakeyama et al., ibid. 318, 467 (1985)

15. W.C. Greene et al., J. Exp. Med. 162, 363 (1985), S. Kondo et al., ibid. 320, 75 (1986); R.J. Robb, Proc.Natl. Acad. SCi.U.S.A. 83, 3992 (1986)

16. M. Sharon, R.D. Klausner, B.R. Cullen, R. Chizzonite, W.J. Leonard, Science 234, 859 (1986)

17. R.J. Robb et al., Proc.Natl.Acad.Sci.U.S.A., 84, 2002 (1987); M. Tsudo, R.W. Kozak, C.K. Goldman, T.A. Waldmann, ibid., p 4215 (1987); M. Dukovich et al., Nature 327, 518 (1987)

18. M. Hatakeyama et al., J.Exp.Med. 166, 362 (1987); S. Kondo et al., Nature 327, 64 (1987)

19. M. Tsudo, F. Kitamura, M. Miyasaka, Proc.Natl.Acad.Sci.U.S.A. in press.

20. J. Yodoi et al., J.Immunol. 134, 1623 (1985)

21. B. Seed, A. Aruffo, Proc.Natl.Acad.Sci.U.S.A. 84, 3365 (1987); B. Seed, Nature 328, 840 (1987)

22. D. Perlman, H.O. Halvorson, J.Mol.Biol. 167, 391 (1983); G. von Heijne, Nucleic Acids Res. 14, 4683 (1986)

23. A. Ullrich et al., Nature 309, 418 (1984); A. Ullrich et al., ibid. 313, 756 (1985); U. Ebina et al., Cell 40, 747 (1985)

24. L. Collins et al., Proc.Natl.Acad.Sci.U.S.A. 85, 7709 (1988)

25. K.S. Hanks, A.M. Quinn, T. Hunter, Science 241, 42 (1988)

26. A. Alcover et al., Immunol.Rev. 95,5 (1987)

27. M. Hatakeyama, S. Minamoto, T. Taniguchi, Proc.Natl.Acad.Sci.U.S.A. 83, 9650 (1986)

28. M. Hatakeyama, T. Doi, T. Kono, S. Minamoto, T. Taniguchi, unpublished observations

29. J.D. Marth, R. Peet, E.G. Krebs, R.M. Perlmutter, Cell 43, 393 (1985)

30. R. Mann, R.C. Mulligan, D. Baltimore, ibid. 33, 153 (1983)

31. M. Fujii et al., J.Exp.Med. 163, 550 (1986)

32. A.M Weissman et al., Proc.Natl.Acad.Sci.U.S.A. 83, 1463 (1986)

33. R.J Robb, W.C. Greene, J.Exp.Med. 165, 1201 (1987)

34. K. Sugamura et 1., ibid. 161, 1243 (1985)

35. H. Saragovi, T.R. Malek, J.Immunol. 141, 476 (1988)

36. J. Kyte, R.F. Doolittle, J.Mol.Biol. 157, 105 (1982)

37. H. Potter, L. Weir, P. Leder, Proc.Natl.Acad.Sci.U.S.A. 81, 7161 (1984)

**Claims**

1. A recombinant DNA molecule coding for the β-chain of an IL-2 receptor or a portion thereof.

2. A recombinant DNA molecule as defined in claim 1 coding for the β-chain of human or murine IL-2 receptor or a portion thereof.

3. A recombinant DNA molecule characterized by a structural gene having the formula:

```
                                                      ATG
GCG GCC CCT GCT CTG TCC TGG CGT CTG CCC CTC CTC ATC
CTC CTC CTG CCC CTG GCT ACC TCT TGG GCA TCT GCA GCG
GTG AAT GGC ACT TCC CAG TTC AGA TGC TTC TAC AAC TCG
AGA GCC AAC ATC TCC TGT CTC TGG AGC CAA GAT GGG GCT
CTG CAG GAC ACT TCC TGC CAA GTC CAT GCC TGG CCG GAC
AGA CGG CGG TGG AAC CAA ACC TGT GAG CTG CTC CCC GTG
AGT CAA GCA TCC TGG GCC TGC AAC CTG ATC CTC GGA GCC
CCA GAT TCT CAG AAA CTG ACC ACA GTT GAC ATC GTC ACC
CTG AGG GTG CTG TGC CGT GAG GGG GTG CGA TGG AGG GTG
ATG GCC ATC CAG GAC TTC AAG CCC TTT GAG AAC CTT CGC
CTG ATG GCC CCC ATC TCC CTC CAA GTT GTC CAC GTG GAG
ACC CAC AGA TGG AAC ATA AGC TGG GAA ATC TCC CAA GCC
TCC CAC TAC TTT GAA AGA CAC CTG GAG TTC GAG GCC CGG
ACG CTG TCC CCA GGC CAC ACC TGG GAG GAG GCC CCC CTG
CTG ACT CTC AAG CAG AAG CAG GAA TGG ATC TGC CTG GAG
ACG CTC ACC CCA GAC ACC CAG TAT GAG TTT CAG GTG CGG
GTC AAG CCT CTG CAA GGC GAG TTC ACG ACC TGG AGC CCC
TGG AGC CAG CCC CTG GCC TTC AGG ACA AAG CCT GCA GCC
CTT GGG AAG GAC ACC ATT CCG TGG CTC GGC CAC CTC CTC
GTG GGC CTC AGC GGG GCT TTT GGC TTC ATC ATC TTA GTG
TAC TTG CTG ATC AAC TGC AGG AAC ACC GGG CCA TGG CTG
AAG AAG CTC CTG AAG TGT AAC ACC CCA GAC CCC TCG AAG
TTC TTT TCC CAG CTG AGC TCA GAG CAT GGA GGA GAC GTC
CAG AAG TGG CTC TCT TCG CCC TTC CCC TGA TCG TCC TTC
```

```
AGC CCT GGC GGC CTG GCA CCT GAG ATC TCG CCA CTA GAA
GTG CTG GAG AGG GAC AAG GTG ACG CAG CTG CTC CTG CAG
CAG GAC AAG GTG CCT GAG CCC GCA TCC TTA AGC AGC AAC
CAC TCG CTG ACC AGC TGC TTC ACC AAC CAG GGT TAC TTC
TTC TTC CAC CTC CCG GAT GCC TTG GAG ATA GAG GCC TGC
CAG GTG TAC TTT ACT TAC GAC CCC TAC TCA GAG GAA GAC
CCT GAT GAG GGT GTG GCC GGG GCA CCC ACA GGG TCT TCC
CCC CAA CCC CTG CAG CCT CTG TCA GGG GAG GAC GAC GCC
TAC TGC ACC TTC CCC TCC AGG GAT GAC CTG CTG CTC TTC
TCC CCC AGT CTC CTC GGT GGC CCC AGC CCC CCA AGC ACT
GCC CCT GGG GGC AGT GGG GCC GGT GAA GAG AGG ATG CCC
CCT TCT TTG CAA GAA AGA GTC CCC AGA GAC TGG GAC CCC
CAG CCC CTG GGG CCT CCC ACC CCA GGA GTC CCA GAC CTG
GTG GAT TTT CAG CCA CCC CCT GAG CTG GTG CTG CGA GAG
GCT GGG GAG GAG GTC CCT GAC GCT GGC CCC AGG GAG GGA
GTC AGT TTC CCC TGG TCC AGG CCT CCT GGG CAG GGG GAG
TTC AGG GCC CTT AAT GCT CGC CTG CCC CTG AAC ACT GAT
GCC TAC TTG TCC CTC CAA GAA CTC CAG GGT CAG GAC CCA
ATC CAC TTG GTG TAG
```

or a portion thereof or a degenerate variant thereof.

4. A recombinant DNA molecule according to claim 3 characterized by a DNA sequence having the formula:

```
                          GCAGCCAGAGCTCAGCAGGGCCCTGGAGAGATGG
CCACGGTCCCAGCACCGGGGAGGACTGGAGAGCGCGCGCTGCCACCGCCCC
ATGTCTCAGCCAGGGCTTCCTTCCTCGGCTCCACCCTGTGGATGTA   ATG
GCG GCC CCT GCT CTG TCC TGG CGT CTG CCC CTC CTC ATC
CTC CTC CTG CCC CTG GCT ACC TCT TGG GCA TCT GCA GCG
GTG AAT GGC ACT TCC CAG TTC AGA TGC TTC TAC AAC TCG
AGA GCC AAC ATC TCC TGT CTC TGG AGC CAA GAT GGG GCT
CTG CAG GAC ACT TCC TGC CAA GTC CAT GCC TGG CCG GAC
AGA CGG CGG TGG AAC CAA ACC TGT GAG CTG CTC CCC GTG
AGT CAA GCA TCC TGG GCC TGC AAC CTG ATC CTC GGA GCC
```

14

CCA GAT TCT CAG AAA CTG ACC ACA GTT GAC ATC GTC ACC
CTG AGG GTG CTG TGC CGT GAG GGG GTG CGA TGG AGG GTG
ATG GCC ATC CAG GAC TTC AAG CCC TTT GAG AAC CTT CGC
CTG ATG GCC CCC ATC TCC CTC CAA GTT GTC CAC GTG GAG
ACC CAC AGA TGG AAC ATA AGC TGG GAA ATC TCC CAA GCC
TCC CAC TAC TTT GAA AGA CAC CTG GAG TTC GAG GCC CGG
ACG CTG TCC CCA GGC CAC ACC TGG GAG GAG GCC CCC CTG
CTG ACT CTC AAG CAG AAG CAG GAA TGG ATC TGC CTG GAG
ACG CTC ACC CCA GAC ACC CAG TAT GAG TTT CAG GTG CGG
GTC AAG CCT CTG CAA GGC GAG TTC ACG ACC TGG AGC CCC
TGG AGC CAG CCC CTG GCC TTC AGG ACA AAG CCT GCA GCC
CTT GGG AAG GAC ACC ATT CCG TGG CTC GGC CAC CTC CTC
GTG GGC CTC AGC GGG GCT TTT GGC TTC ATC ATC TTA GTG
TAC TTG CTG ATC AAC TGC AGG AAC ACC GGG CCA TGG CTG
AAG AAG CTC CTG AAG TGT AAC ACC CCA GAC CCC TCG AAG
TTC TTT TCC CAG CTG AGC TCA GAG CAT GGA GGA GAC GTC
CAG AAG TGG CTC TCT TCG CCC TTC CCC TGA TCG TCC TTC
AGC CCT GGC GGC CTG GCA CCT GAG ATC TCG CCA CTA GAA
GTG CTG GAG AGG GAC AAG GTG ACG CAG CTG CTC CTG CAG
CAG GAC AAG GTG CCT GAG CCC GCA TCC TTA AGC AGC AAC
CAC TCG CTG ACC AGC TGC TTC ACC AAC CAG GGT TAC TTC
TTC TTC CAC CTC CCG GAT GCC TTG GAG ATA GAG GCC TGC
CAG GTG TAC TTT ACT TAC GAC CCC TAC TCA GAG GAA GAC
CCT GAT GAG GGT GTG GCC GGG GCA CCC ACA GGG TCT TCC
CCC CAA CCC CTG CAG CCT CTG TCA GGG GAG GAC GAC GCC
TAC TGC ACC TTC CCC TCC AGG GAT GAC CTG CTG CTC TTC
TCC CCC AGT CTC CTC GGT GGC CCC AGC CCC CCA AGC ACT
GCC CCT GGG GGC AGT GGG GCC GGT GAA GAG AGG ATG CCC
CCT TCT TTG CAA GAA AGA GTC CCC AGA GAC TGG GAC CCC
CAG CCC CTG GGG CCT CCC ACC CCA GGA GTC CCA GAC CTG
GTG GAT TTT CAG CCA CCC CCT GAG CTG GTG CTG CGA GAG
GCT GGG GAG GAG GTC CCT GAC GCT GGC CCC AGG GAG GGA
GTC AGT TTC CCC TGG TCC AGG CCT CCT GGG CAG GGG GAG
TTC AGG GCC CTT AAT GCT CGC CTG CCC CTG AAC ACT GAT
GCC TAC TTG TCC CTC CAA GAA CTC CAG GGT CAG GAC CCA

```
ATC CAC TTG GTG TAG   ACAGATGGCCAGGGTGGGAGGCAGGCAGCT
GCCTGCTCTGCGCCGAGCCTCAGAAGGACCCTGTTGAGGGTCCTCAGTCCA
CTGCTGAGGACACTCAGTGTCCAGTTGCAGCTGGACTTCTCCACCCGGATG
GCCCCCACCCAGTCCTGCACACTTGGTCCATCCATTTCCAAACCTCCACTG
CTGCTCCCGGGTCCTGCTGCCCGAGCCAGGAACTGTGTGTGTTGCAGGGGG
GCAGTAACTCCCCAACTCCCTCGTTAATCACAGGATCCCACGAATTTAGGC
TCAGAAGCATCGCTCCTCTCCAGCCCTGCAGCTATTCACCAATATCAGTCC
TCGCGGCTCTCCAGGGCTCCCTGCCCTGACCTCTTCCCTGGGTTTTCTGCC
CCAGCCTCCTCCTTCCCTCCCCTCCCCGTCCACAGGGCAGCCTGAGCGTGC
TTTCCAAAACCCAAATATGGCCACGCTCCCCCTCGGTTCAAAACCTTGCAC
AGGTCCCACTGCCCTCAGCCCCACTTCTCAGCCTGGTACTTGTACCTCCGG
TGTCGTGTGGGGACATCCCCTTCTGCAATCCTCCCTACCGTCCTCCCGAGC
CACTCAGAGCTCCCTCACACCCCCTCTGTTGCACATGCTATTCCCTGGGGC
TGCTGTGCGCTCCCCCTCATCTAGGTGACAAACTTCCCTGACTCTTCAAGT
GCCGGTTTTGCTTCTCCTGGAGGGAAGCACTGCCTCCCTTAATCTGCCAGA
AACTTCTAGCGTCAGTGCTGGAGGGAGAAGCTGTCAGGGACCCAGGGCGCC
TGGAGAAAGAGGCCCTGTTACTATTCCTTTGGGATCTCTGAGGCCTCAGAG
TGCTTGGCTGCTGTATCTTTAATGCTGGGGCCCAAGTAAGGGCACAGATCC
CCCCGACAAAGTGGATGCCTGCTGCATCTTCCCACAGTGGCTTCACAGACC
CACAAGAGAAGCTGATGGGGAGTAAACCCTGGAGTCCGAGGCCCAGGCAGC
AGCCCCGCCTAGTGGTGGGCCCTGATGCTGCCAGGCCTGGGACCTCCCACT
GCCCCCTCCACTGGAGGGGTCTCCTCTGCAGCTCAGGGACTGGCACACTGG
CCTCCAGAAGGGCAGCTCCACAGGGCAGGGCCTCATTATTTTTCACTGCCC
CAGACACAGTGCCCAACACCCCGTCGTATACCCTGGATGAACGAATTAATT
ACCTGGCACCACCTCGTCTGGGCTCCCTGCGCCTGACATTCACACAGAGAG
GCAGAGTCCCGTGCCCATTAGGTCTGGCATGCCCCCTCCTGCAAGGGGCTC
AACCCCCTACCCCGACCCCTCCACGTATCTTTCCTAGGCAGATCACGTTGC
AATGGCTCAAACAACATTCCACCCCAGCAGGACAGTGACCCCAGTCCCAGC
TAACTCTGACCTGGGAGCCCTCAGGCACCTGCACTTACAGGCCTTGCTCAC
AGCTGATTGGGCACCTGACCACACGCCCCCACAGGCTCTGACCAGCAGCCT
ATGAGGGGGTTTGGCACCAAGCTCTGTCCAATCAGGTAGGCTGGGCCTGAA
CTAGCCAATCAGATCAACTCTGTCTTGGGCGTTTGAACTCAGGGAGGGAGG
CCCTTGGGAGCAGGTGCTTGTGGACAAGGCTCCACAAGCGTTGAGCCTTGG
AAAGGTAGACAAGCGTTGAGCCACTAAGCAGAGGACCTTGGGTTCCCAATA
CAAAAATACCTACTGCTGAGAGGGCTGCTGACCATTTGGTCAGGATTCCTG
```

```
TTGCCTTTATATCCAAAATAAACTCCCCTTTCTTGAGGTTGTCTGAGTCTT
GGGTCTATGCCTTGAAAAAAGCTGAATTATTGGACAGTCTCACCTCCTGCC
ATAGGGTCCTGAATGTTTCAGACCACAAGGGGCTCCACACCTTTGCTGTGT
GTTCTGGGGCAACCTACTAATCCTCTCTGCAAGTCGGTCTCCTTATCCCCC
CAAATGGAAATTGTATTTGCCTTCTCCACTTTGGGAGGCTCCCACTTCTTG
GGAGGGTTACATTTTTTAAGTCTTAATCATTTGTGACATATGTATCTATAC
ATCCGTATCTTTTAATGATCCGTGTGTACCATCTTTGTGATTATTTCCTTA
ATATTTTTTCTTTAAGTCAGTTCATTTTCGTTGAAATACATTTATAAAGAA
AAATCTTTGTTACTCTGTAAATGAAAAAACCCATTTTCGCTATAAATAAAA
GGTAACTGTACAAAATAAGTACAAT
```

or a portion thereof or a degenerate variant thereof.

5. A recombinant DNA molecule according to claim 1 characterized by a structural gene having the formula

EP 0 386 304 A1

ATG

GCT ACC ATA GCT CTT CCC TGG AGC CTG TCC CTC TAC GTC TTC CTC CTG CTC CTG GCT ACA CCT TGG GCA TCT GCA

GCA GTG AAA AAC TGT TCC CAT CTT GAA TGC TTC TAC AAC TCA AGA GCC AAT GTC TCT TGC ATG TGG AGC CAT GAA

GAG GCT CTG AAT GTC ACA ACC TGC CAC GTC CAT GCC AAG TCG AAC CTG CGA CAC TGG AAC AAA ACC TGT GAG CTA

ACT CTT GTG AGG CAG GCA TCC TGG GCC TGC AAC CTG ATC CTC GGG TCG TTC CCA GAG TCC CAG TCA CTG ACC TCC

GTG GAC CTC CTT GAC ATA AAT GTG GTG TGC TGG GAA GAG AAG GGT TGG CGT AGG GTA AAG ACC TGC GAC TTC CAT

CCC TTT GAC AAC CTT CGC CTG GTG GCC CCT CAT TCC CTC CAA GTT CTG CAC ATT GAT ACC CAG AGA TGT AAC ATA

AGC TGG AAG GTC TCC CAG GTC TCT CAC TAC ATT GAA CCA TAC TTG GAA TTT GAG GCC CGT AGA CGT CTT CTG GGC

CAC AGC TGG GAG GAT GCA TCC GTA TTA AGC CTC AAG CAG AGA CAG ACG TGG CTC TTC TTG GAG ATG CTG ATC CCT

AGT ACC TCA TAT GAG GTC CAG GTG AGG GTC AAA GCT CAA CGA AAC AAT ACC GGG ACC TGG AGT CCC TGG AGC CAG

CCC CTG ACC TTT CGG ACA AGG CCA GCA GAT CCC ATG AAG GAG ATC CTC CCC ATG TCA TGG CTC AGA TAC CTT CTG

CTG GTC CTT GGT TGT TTT TCT GGC TTC TTC TCC TGC GTC TAC ATT TTG GTC AAG TGC CGG TAC CTT GGG CCA TGG

```
CTG AAG ACA GTT CTC AAG TGC CAC ATC CCA GAT CCT TCT GAG TTC TTC TCC CAG CTG AGC TCC CAG CAT GGG GGA

GAC CTT CAG AAA TGG CTC TCC TCG CCT GTC CCC TTG TCC TTC TTC AGC CCC AGT GGC CCT GCC CCT GAG ATC TCT

CCG CTG GAA GTG CTC GAC GGA GAT TCC AAG GCC GTG CAG CTG CTC CTG TTA CAG GAC TCT GCC CCT TTA CCC

TCG CCC AGC GGC CAC TCA CAG GCC AGC TGC TTC ACC AAC CAG GGC TAC TTC TTC CAT CTG CCC AAT GCC TTG

GAG ATC GAA TCC TGC CAG GTG TAC TTC ACC TAT GAC CCC TGT GTG GAA GAG GAG GTG GAG GAT GGG TCA AGG

CTG CCC GAG TCT CCC CAC CCA CCT CTG GCT CTG CCT CTG GCT GGA GAA CAG GAT GAC TAC TGT GCC TTC CCG CCC

AGG GAT GAC CTG CTC TTC TCC CCG AGC CTC AGC ACC CCC AAC ACT GCC TAT GGG GGC AGC AGA GCC CCT GAA

GAA AGA TCT CCA CTC TCC CTG CAT GAG GGT GAT GGA GAG GGG CTT CCC TCC CTA GCA TCC CGT GAC CTG ATG GGC TTA CAG CGC CCT

CTG GAG CGG ATG CCG GAA GGT GAT GGA GAG GGG CTG TCT GCC AAT AGC TCT GGG GAG CAG GCC AGT GTC CCA GAA

GGC AAC CTT CAT GGG CAA GAT CAG GAC AGA GGC CAG GCC CCC ATC CTG ACC CTG AAC ACC GAT GCC TAT CTG TCT

CTT CAA GAA CTA CAG GCC CAA GAT TCA GTC CAC CTA ATA TAG ***
```

or a degenerate variant thereof.

6. A recombinant DNA molecule according to claim 1 characterized by an DNA sequence having the

formula:

CGTTTCTCTCTCTTGCTCTCTTGCTTCTTGCT
GAAGATTCTGGTCTGTGGTGTTCTTCCTGGCCGGTCGTGTGAATAACAATTGGTGCCGAAACCCGGGACGAGAAAAAACTCGGGACGAGA
GTTTACATCACCGGTGCAAGGAAGATCCCTCATTCCAGAAGAACCAGAACTGCGGGTCGGTAATAAAGACTGTTAAGAAGGATTC
AACTGTATGAATTCAGAACTTTCAGCTGGGAACCTGGGAACGAGAGATCCAGTGAGTACAGAGCTTTACGAGGGTTTGCATCCTCAGCTCCTCAGCTCTGTG   ATG

GCT ACC ATA GCT CTT CCC TGG AGC CTG TCC CTC TAC GTC TTC CTC CTG GCT ACA CCT TGG GCA TCT GCA

GCA GTG AAA AAC TGT TCC CAT CTT GAA TGC TTC TAC AAC TCA AGA GCC AAT GTC TCT TGC ATG TGG AGC CAT GAA

GAG GCT CTG AAT GTC ACA ACC TGC CAC GTC CGA CAC TGG AAC AAA ACC TGT GAG CTA

ACT CTT GTG AGG CAG GCA TCC TGG GCC TGC AAC CTG ATC CTC GGG TCG TTC CCA GAG TCA CTG ACC TCC

GTG GAC CTC CTT GAC AAT GTG GTG TGC GCC CCT CAT TCC CTC CAA GTT CTG CAC ATT GAT ACC CAG AGA TGT AAC ATA

CCC TTT GAC AAC CTT CGC CTG GTG CAG GTC TCT CAC TAC ATT GAA TTT GAG GCC CGT AGA CGT CTT CTG GGC

AGC TGG AAG GTC TCC CAG GTC GCA TCC GTA TTA AGC CTC AAG CAG ACG TGG GAG ATG CTG ATC CCT

CAC AGC TGG GAG GAT GCA TCC CAG GTG AGG GTC AAA GCT CAA CGA AAC AAT ACC GGG AGT CCC TGG AGC CAG

AGT ACC TCA TAT GAG GTC CAG GTG AGG CCA GCA GAT CCC AAG GAG ATC CTC CCC ATG TCA TGG CTC AGA TAC CTT CTG

CCC CTG ACC TTT CGG ACA AGG CCA GAT CCC TTC TTC TGC TAC ATT TTG GTC TAC CTT GGG TAC CTT GGG CCA TGG

CTG GTC CTT GGT TGT TTT TCT GGC TTC TTC TCC GTC GTC TAC

```
CTG AAG ACA GTT CTC AAG TGC CAC ATC CCA GAT CCT TCT GAG TTC TTC TCC CAG CTG AGC TCC CAG CAT GGG GGA
GAC CTT CAG AAA TGG CTC TCC TCG CCT GTC CCC TTG TCC TTC TTC AGC CCC AGT GGC CCT GCC ATC TCT
CCG CTG GAA GTG CTC CTC GAC GGA GAT TCC AAG GCC GTG CAG CTG TTA CAG AAG GAC TCT GCC CCT TTA CCC
TCG CCC AGC GGC CAC TCA CAG GCC AGC TGC TTC ACC AAC CAG GGC TAC TTC TTC CAT CTG CCC AAT GCC TTG
GAG ATC GAA TCC TGC CAG GTG TAC TTC ACC TAT GAC CCC TGT GTG GAA GAG GAG GTG GAG GAT GGG TCA AGG
CTG CCC GAG GGA TCT CCC CAC CCA CCT CTG CCT CTG GCT GGA GAA CAG GAT GAC TAC TGT GCC TTC CCG CCC
AGG GAT GAC CTG CTC TTC TCC CCG AGC CTC AGC ACC CCC AAC ACT GCC TAT GGG GGC AGC AGA GCC CCT GAA
GAA AGA TCT CCA CTC CTC TCC CTG CAT GAG GGA GGT GAT GGA GAG GGG CTG TCT GCC AAT AGC TCT GGG GAG CAG GCC CCT
CTG GAG CGG ATG CCG GAA GGT GAT GGA GAG GGG CTG TCT GCC AAT AGC TCT GGG GAG CAG GCC AGT GTC CCA GAA
GGC AAC CTT CAT GGG CAA GAT CAG GCC CAA GAT GCC ATC CTG AAC ACC GAT GCC TAT CTG TCT
CTT CAA GAA CTA CAG GCC CAA GAT TCA GTC CAC CTA ATA TAG   CAGGTGGCCAGGACTGGGATCCAGCTGCCTGGATCAGGTCAG
                                                * * *

GCTTGAGGAAGACTGCTTAAGAGGTCTCTTGAGGACAGTCCACTGCTGAGGACTGTCCTGAGGACTGTCCTGGCTATCCCTGCCCCCCCCTCCAAACTTAATCATCCACT
TCTGAACTCCATTTGCTACTTCCTGGTCTAACCAGGTTTGGTGGAGGGTGGGGAGTGGGGGAGCGGTGGTCAGCTCCCACTGCCCTATTTAGTCATGAG
GTCACTAGCTTCCTGTACCTGCCTCCTTCCCTCCTTCCCTGCCTTCTTCACAGGGCAGCTAGAACTTGCTTCCCCG
```

or a degenerate variant thereof.

7. A recombinant DNA molecule as defined in any one of claims 1 to 6 which further comprises regulatory sequences operably linked to the structural gene for the IL-2β chain or portion thereof.

8. A recombinant DNA molecule as defined in claim 7 which is a plasmid.

9. A recombinant DNA molecule as defined in claim 8, this being one of the following

pIL-2Rβ6,

pIL-2Rβ9,

pIL-2Rβ19,

pIL-2Rβ30.

pMIL-2Rβ 36

10. A host cell which has been transformed by a recombinant DNA molecule as defined in any one of claims 1 to 9.

11. A host cell as defined in claim 10, which is a bacterial cell or a yeast cell or a mammalian cell.

12. A protein having the structure defined by the structural gene set forth in claim 2 to 5a portion thereof.

13. A hybridoma, sub-clone or mutant thereof capable of secreting a monoclonal antibody having a specific affinity to a protein as defined in claim 12.

14. A monoclonal antibody having a specific affinity to a protein as defined in claim 12.

15. A method of producing a hybridoma as defined in claim 13 which comprises immunizing a non-human animal with a protein as defined in claim 9, removing spleen cells from the immunized animal and fusing the spleen cells with non-immunoglobulin secreting myloma cells, and selecting from the resulting hybridomas a cell line which produces a monoclonal antibody having the desired binding specificity and, if desired, subsequently sub-cloning said hybridoma.

FIG. 1A

Fig. 1 B

Page 1)

```
                         GCAGCCAGAGCTCAGCAGGGCCCTGGAGAGATGG        34

        CCACGGTCCCAGCACCGGGGAGGACTGGAGAGCGCGCGCTGCCACCGCCCC

        ATGTCTCAGCCAGGGCTTCCTTCCTCGGCTCCACCCTGTGGATGTA   ATG   134
                                                       Met
                                                       -26

     GCG GCC CCT GCT CTG TCC TGG CGT CTG CCC CTC CTC ATC    173
-25  Ala Ala Pro Ala Leu Ser Trp Arg Leu Pro Leu Leu Ile

     CTC CTC CTG CCC CTG GCT ACC TCT TGG GCA TCT GCA GCG    212
-12  Leu Leu Leu Pro Leu Ala Thr Ser Trp Ala Ser Ala Ala

     GTG AAT GGC ACT TCC CAG TTC AGA TGC TTC TAC AAC TCG    251
  2  Val Asn Gly Thr Ser Gln Phe Thr Cys Phe Tyr Asn Ser

     AGA GCC AAC ATC TCC TGT CTC TGG AGC CAA GAT GGG GCT    290
 15  Arg Ala Asn Ile Ser Cys Val Trp Ser Gln Asp Gly Ala

     CTG CAG GAC ACT TCC TGC CAA GTC CAT GCC TGG CCG GAC    329
 28  Leu Gln Asp Thr Ser Cys Gln Val His Ala Trp Pro Asp

     AGA CGG CGG TGG AAC CAA ACC TGT GAG CTG CTC CCC GTG    368
 41  Arg Arg Arg Trp Asn Gln Thr Cys Glu Leu Leu Pro Val

     AGT CAA GCA TCC TGG GCC TGC AAC CTG ATC CTC GGA GCC    407
 54  Ser Gln Ala Ser Trp Ala Cys Asn Leu Ile Leu Gly Ala

     CCA GAT TCT CAG AAA CTG ACC ACA GTT GAC ATC GTC ACC    446
 67  Pro Asp Ser Gln Lys Leu Thr Thr Val Asp Ile Val Thr

     CTG AGG GTG CTG TGC CGT GAG GGG GTG CGA TGG AGG GTG    485
 80  Leu Arg Val Leu Cys Arg Glu Gly Val Arg Trp Arg Val

     ATG GCC ATC CAG GAC TTC AAG CCC TTT GAG AAC CTT CGC    524
 93  Met Ala Ile Gln Asp Phe Lys ProPhe Glu Asn Leu Arg

     CTG ATG GCC CCC ATC TCC CTC CAA GTT GTC CAC GTG GAG    563
106  Leu Met Ala Pro Ile Ser Leu Gln Val Val His Val Glu

     ACC CAC AGA TGG AAC ATA AGC TGG GAA ATC TCC CAA GCC    602
119  Thr His Arg Cys Asn Ile Ser Trp Glu Ile Ser Gln Ala

     TCC CAC TAC TTT GAA AGA CAC CTG GAG TTC GAG GCC CGG    641
132  Ser His Tyr Phe Glu Arg His Leu Glu Phe Glu Ala Arg

     ACG CTG TCC CCA GGC CAC ACC TGG GAG GAG GCC CCC CTG    680
145  Thr Leu Ser Pro Gly His Thr Trp Glu Glu Ala Pro Leu
```

Fig. 1 B

EP 0 386 304 A1

Page 2)

```
        CTG ACT CTC AAG CAG AAG CAG GAA TGG ATC TGC CTG GAG    719
158     Leu Thr Leu Lys Gln Lys Gln Glu Trp Ile Cys Leu Glu

        ACG CTC ACC CCA GAC ACC CAG TAT GAG TTT CAG GTG CGG    758
171     Thr Leu Thr Pro Asp Thr Gln Tyr Glu Phe Gln Val Arg

        GTC AAG CCT CTG CAA GGC GAG TTC ACG ACC TGG AGC CCC    797
184     Val Lys Pro Leu Gln Gly Glu Phe Thr Thr Trp Ser Pro

        TGG AGC CAG CCC CTG GCC TTC AGG ACA AAG CCT GCA GCC    836
197     Trp Ser Gln Pro Leu Ala Phe Arg Thr Lys Pro Ala Ala

        CTT GGG AAG GAC ACC ATT CCG TGG CTC GGC CAC CTC CTC    875
210     Leu Gly Lys Asp Thr Ile Pro Trp Leu Gly His Leu Leu

        GTG GGC CTC AGC GGG GCT TTT GGC TTC ATC ATC TTA GTG    914
223     Val Gly Leu Ser Gly Ala Phe Gly Phe Ile Ile Leu Val

        TAC TTG CTG ATC AAC TGC AGG AAC ACC GGG CCA TGG CTG    953
236     Thr Leu Leu Ile Asn Cys Arg Asn Thr Gly Pro Trp Leu

        AAG AAG CTC CTG AAG TGT AAC ACC CCA GAC CCC TCG AAG    992
249     Lys Lys Val Leu Lys Cys Asn Thr Pro Asp Pro Ser Lys

        TTC TTT TCC CAG CTG AGC TCA GAG CAT GGA GGA GAC GTC    1031
262     Phe Phe Ser Gln Leu Ser Ser Glu His Gly Gly Asp Val

        CAG AAG TGG CTC TCT TCG CCC TTC CCC TGA TCG TCC TTC    1070
275     Gln Lys Trp Leu Ser Ser Pro Phe Pro Ser Ser Ser Phe

        AGC CCT GGC GGC CTG GCA CCT GAG ATC TCG CCA CTA GAA    1109
288     Ser Pro Gly Gly Leu Ala Pro Glu Ile Ser Pro Leu Glu

        GTG CTG GAG AGG GAC AAG GTG ACG CAG CTG CTC CTG CAG    1148
301     Val Leu Glu Arg Asp Lys Val Thr Gln Leu Leu Leu Gln

        CAG GAC AAG GTG CCT GAG CCC GCA TCC TTA AGC AGC AAC    1187
314     Gln Asp Lys Val Pro Glu Pro Ala Ser Leu Ser Ser Asn

        CAC TCG CTG ACC AGC TGC TTC ACC AAC CAG GGT TAC TTC    1226
327     His Ser Leu Thr Ser Cys Phe Thr Asn Gln Gly Tyr Phe

        TTC TTC CAC CTC CCG GAT GCC TTG GAG ATA GAG GCC TGC    1265
340     Phe Phe His Leu Pro Asp Ala Leu Glu Ile Glu Ala Cys

        CAG GTG TAC TTT ACT TAC GAC CCC TAC TCA GAG GAA GAC    1304
353     Gln Val Tyr Phe Thr Tyr Asp Pro Tyr Ser Glu Glu Asp

        CCT GAT GAG GGT GTG GCC GGG GCA CCC ACA GGG TCT TCC    1343
366     Pro Asp Glu Gly Val Ala Gly Ala Pro Thr Gly Ser Ser

        CCC CAA CCC CTG CAG CCT CTG TCA GGG GAG GAC GAC GCC    1382
379     Pro Gln Pro Leu Gln Pro Leu Ser Gly Glu Asp Asp Ala
```

Fig. 1 B

EP 0 386 304 A1

Page 3)

```
     TAC TGC ACC TTC CCC TCC AGG GAT GAC CTG CTG CTC TTC     1421
392  Tyr Cys Thr Phe Pro Ser Arg Asp Asp Leu Leu Leu Phe

     TCC CCC AGT CTC CTC GGT GGC CCC AGC CCC CCA AGC ACT     1460
405  Ser Pro Ser Leu Leu Gly Gly Pro Ser Pro Pro Ser Thr

     GCC CCT GGG GGC AGT GGG GCC GGT GAA GAG AGG ATG CCC     1499
418  Ala Pro Gly Gly Ser Gly Ala Gly Glu Glu Arg Met Pro

     CCT TCT TTG CAA GAA AGA GTC CCC AGA GAC TGG GAC CCC     1538
431  Pro Ser Leu Gln Glu Arg Val Pro Arg Asp Trp Asp Pro

     CAG CCC CTG GGG CCT CCC ACC CCA GGA GTC CCA GAC CTG     1577
444  Gln Pro Leu Gly Pro Pro Thr Pro Gly Val Pro Asp Leu

     GTG GAT TTT CAG CCA CCC CCT GAG CTG GTG CTG CGA GAG     1616
457  Val Asp Phe Gln Pro Pro Pro Glu Leu Val Leu Arg Glu

     GCT GGG GAG GAG GTC CCT GAC GCT GGC CCC AGG GAG GGA     1655
470  Ala Gly Glu Glu Val Pro Asp Ala Gly Pro Arg Glu Gly

     GTC AGT TTC CCC TGG TCC AGG CCT CCT GGG CAG GGG GAG     1694
483  Val Ser Phe Pro Trp Ser Arg Pro Pro Gly Gln Gly Glu

     TTC AGG GCC CTT AAT GCT CGC CTG CCC CTG AAC ACT GAT     1733
496  Phe Arg Ala Leu Asn Ala Arg Leu Pro Leu Asn Thr Asp

     GCC TAC TTG TCC CTC CAA GAA CTC CAG GGT CAG GAC CCA     1772
509  Ala Tyr Leu Ser Leu Gln Glu Leu Gln Gly Gln Asp Pro

     ATC CAC TTG GTG TAG  ACAGATGGCCAGGGTGGGAGGCAGGCAGCT     1817
522  Thr His Leu Val ***
```

```
GCCTGCTCTGCGCCGAGCCTCAGAAGGACCCTGTTGAGGGTCCTCAGTCCA
CTGCTGAGGACACTCAGTGTCCAGTTGCAGCTGGACTTCTCCACCCGGATG
GCCCCCACCCAGTCCTGCACACTTGGTCCATCCATTTCCAAACCTCCACTG
CTGCTCCCGGGTCCTGCTGCCCGAGCCAGGAACTGTGTGTGTTGCAGGGGG
GCAGTAACTCCCCAACTCCCTCGTTAATCACAGGATCCCACGAATTTAGGC
TCAGAAGCATCGCTCCTCTCCAGCCCTGCAGCTATTCACCAATATCAGTCC
TCGCGGCTCTCCAGGGCTCCCTGCCCTGACCTCTTCCCTGGGTTTTCTGCC
CCAGCCTCCTCCTTCCCTCCCCTCCCCGTCCACAGGGCAGCCTGAGCGTGC
TTTCCAAAACCCAAATATGGCCACGCTCCCCCTCGGTTCAAAACCTTGCAC
AGGTCCCACTGCCCTCAGCCCCACTTCTCAGCCTGGTACTTGTACCTCCGG
TGTCGTGTGGGGACATCCCCTTCTGCAATCCTCCCTACCGTCCTCCCGAGC
CACTCAGAGCTCCCTCACACCCCCTCTGTTGCACATGCTATTCCCTGGGGC
TGCTGTGCGCTCCCCCTCATCTAGGTGACAAACTTCCCTGACTCTTCAAGT
```

Fig. 1 B

Page 4)

EP 0 386 304 A1

```
GCCGGTTTTGCTTCTCCTGGAGGGAAGCACTGCCTCCCTTAATCTGCCAGA
AACTTCTAGCGTCAGTGCTGGAGGGAGAAGCTGTCAGGGACCCAGGGCGCC
TGGAGAAAGAGGCCCTGTTACTATTCCTTTGGGATCTCTGAGGCCTCAGAG
TGCTTGGCTGCTGTATCTTTAATGCTGGGGCCCAAGTAAGGGCACAGATCC
CCCCGACAAAGTGGATGCCTGCTGCATCTTCCCACAGTGGCTTCACAGACC
CACAAGAGAAGCTGATGGGGAGTAAACCCTGGAGTCCGAGGCCCAGGCAGC
AGCCCCGCCTAGTGGTGGGCCCTGATGCTGCCAGGCCTGGGACCTCCCACT
GCCCCCTCCACTGGAGGGGTCTCCTCTGCAGCTCAGGGACTGGCACACTGG
CCTCCAGAAGGGCAGCTCCACAGGGCAGGGCCTCATTATTTTTCACTGCCC
CAGACACAGTGCCCAACACCCCGTCGTATACCCTGGATGAACGAATTAATT
ACCTGGCACCACCTCGTCTGGGCTCCCTGCGCCTGACATTCACACAGAGAG
GCAGAGTCCCGTGCCCATTAGGTCTGGCATGCCCCCTCCTGCAAGGGGCTC
AACCCCCTACCCCGACCCCTCCACGTATCTTTCCTAGGCAGATCACGTTGC
AATGGCTCAAACAACATTCCACCCCAGCAGGACAGTGACCCCAGTCCCAGC
TAACTCTGACCTGGGAGCCCTCAGGCACCTGCACTTACAGGCCTTGCTCAC
AGCTGATTGGGCACCTGACCACACGCCCCCACAGGCTCTGACCAGCAGCCT
ATGAGGGGGTTTGGCACCAAGCTCTGTCCAATCAGGTAGGCTGGGCCTGAA
CTAGCCAATCAGATCAACTCTGTCTTGGGCGTTTGAACTCAGGGAGGGAGG
CCCTTGGGAGCAGGTGCTTGTGGACAAGGCTCCACAAGCGTTGAGCCTTGG
AAAGGTAGACAAGCGTTGAGCCACTAAGCAGAGGACCTTGGGTTCCCAATA
CAAAAATACCTACTGCTGAGAGGGCTGCTGACCATTTGGTCAGGATTCCTG
TTGCCTTTATATCCAAAATAAACTCCCCTTTCTTGAGGTTGTCTGAGTCTT
GGGTCTATGCCTTGAAAAAAGCTGAATTATTGGACAGTCTCACCTCCTGCC
ATAGGGTCCTGAATGTTTCAGACCACAAGGGGCTCCACACCTTTGCTGTGT
GTTCTGGGGCAACCTACTAATCCTCTCTGCAAGTCGGTCTCCTTATCCCCC
CAAATGGAAATTGTATTTGCCTTCTCCACTTTGGGAGGCTCCCACTTCTTG
GGAGGGTTACATTTTTTAAGTCTTAATCATTTGTGACATATGTATCTATAC
ATCCGTATCTTTTAATGATCCGTGTGTACCATCTTTGTGATTATTTCCTTA
ATATTTTTTCTTTAAGTCAGTTCATTTTCGTTGAAATACATTTATAAAGAA
AAATCTTTGTTACTCTGTAAATGAAAAAACCCATTTTCGCTATAAATAAAA
GGTAACTGTACAAAATAAGTACAAT
```
                                                                    4034

FIG. 2

(cell length:19)

IL-2Rα

IL-2Rβ

Hydropathy index

Amino acids

EP 0 386 304 A1

# FIG. 3a

# FIG. 3b

# FIG.4a

Relative cell number

(1) ELβ-13  (2) Jβ-8  (3) Jα-5
(4) Jαβ-2  (5) Jαβ-10  (6) Fβ-3

Relative fluorescence intensity (log scale)

Bound IL-2/free IL-2

(1) ELβ-13 [kd : 4.0nM, 4.100 sites/cell]
(2) Jβ-8 [kd : 2.7nM, 1.900 sites/cell]
(3) Jα-5 [kd : 19.5nM, 18.300 sites/cell]
(4) Jαβ-2 [kd : 22pM, 1.250 sites/cell][kd : 15nM, 13.500 sites/cell]
(5) Jαβ-10 [kd : 19pM, 400 sites/cell][kd : 33nM, 12.500 sites/cell]

Bound IL-2 molecules per cell (×10⁻³)

# FIG.4b

FIG.5

# FIG. 6

Fig. 7    Restriction enzyme leavage map of the mouse IL-2
receptor ß chain cDNA clone

EP 0 386 304 A1

Fig. 8

```
CGTTTTCTCTCTCTCTTGCTTCTTCTTACACGCTTGCTCCTGAAGATGTAAGAAATAAAGCTTTGCCCGCA                          82
GAAGATTCTGGTCTGTGGTGTTCTTCCTGGCCGGTCGTGAGAACGCGTCTAATAACAATTGGTGCCGGACGAGAAACCCGGACGAGAAAAAACTCGGGACGAGA   181
GTTTACATCACCGGTGCAAGGAAGAATCCCTCATTCCAGAACCAGAACTGCGGGTAATAAAGGTTCCGTAAAGCAGACTGTTAAGAAGGATTC              280
AACTGTATGAATTCAGAACTTTCAGCTGGGAACGTGAGTACAGAGATCCAGTAGAGAGGTTTGCATCCTCAGCTCCTCAGCTGTG · ATG               377
                                                                                         Met
```

```
  1   GCT ACC ATA GCT CTT CCC TGG AGC CTG TCC CTC TAC GTC TTC CTC CTG GCT ACA CCT TGG GCA TCT GCA    452
      Ala Thr Ile Ala Leu Pro Trp Ser Leu Ser Leu Tyr Val Phe Leu Leu Leu Ala Thr Pro Trp Ala Ser Ala

 26   GCA GTG AAA AAC TGT CTT GAA TGC TTC TTC TCA AGA GCC AAT GTC TCT TGC ATG TGG AGC CAT GAA         527
      Ala Val Lys Asn Cys Leu Glu Cys Phe Phe Ser Arg Ala Asn Val Ser Cys Met Trp Ser His Glu

 51   GAG GCT AAT GTC CTG TGC CAC GTC ACC AAC TGG CGA AAA ACC TGT GAG CTA                             602
      Glu Ala Asn Val Leu Cys His Val Thr Asn Trp Arg Lys Thr Cys Glu Leu

 76   ACT CTT GTG AGG CAG GCA TCC TGG TGG CTG GGG ATC CTC CTC CCA GAG TCA CTG ACC TCC                 677
      Thr Leu Val Arg Gln Ala Ser Trp Trp Leu Gly Ile Leu Leu Pro Glu Ser Leu Thr Ser

101   GTG GAC CTC CTT GAC ATA AAT GTG GTG TGG AAG GAG GTG CGT AGG ACC TGC CAT         752
      Val Asp Leu Leu Asp Ile Asn Val Val Trp Lys Glu Val Arg Arg Thr Cys His

126   CCC TTT GAC AAC CTT CGC GTG GCC CTG GTG CAC GTT CTC CTC ATT GAT TGT ATA         827
      Pro Phe Asp Asn Leu Arg Val Ala Leu Val His Val Leu Leu Ile Asp Cys Asn Ile

151   AGC TGG AAG GTC TCC CAG GTC AGC CTG GTG AAG TTT GAA CCA TAC CTG AGA CGT CTT         902
      Ser Trp Lys Val Ser Gln Val Ser Leu Val Lys Phe Glu Pro Tyr Leu Arg Arg Leu

176   CAC AGC GAT GCA TCC GTA TTA AGC CTC AAG CAG AGA ACG CTC TTG GAG ATG CCT         977
      His Ser Asp Ala Ser Val Leu Ser Leu Lys Gln Arg Thr Leu Phe Leu Glu Met Ile Pro

201   AGT ACC TCA TAT GAG TCA GTG AAG GTC CAA AAA GCT AAC CGA ACC TGG TGG AGC CAG        1052
      Ser Thr Ser Tyr Glu Ser Val Gln Val Lys Ala Asn Arg Thr Gly Trp Ser Gln

226   CCC ACC TTT CGG ACA AGG CCA GAT GAG ATC ATG AAG TAC CTC CTG AGA TAC TTG CTG        1127
      Pro Thr Phe Arg Thr Arg Pro Asp Glu Ile Met Lys Tyr Leu Arg Arg Leu Leu

      CTG GTC CTT TGT TCC GGT GTC TGC TAC TTT GTC AAG TGG CGG CCA TGG        1202
      Leu Val Leu Cys Ser Gly Phe Phe Val Lys Val Tyr Cys Arg Leu Gly Pro Trp
```

con. Fig. 8

251 | CTG AAG ACA GTT CTC AAG TGC CAC ATC CCA GAT CCT TCT GAG TTC TTC TCC CAG CTG AGC TCC CAG CAT GGG GGA | 1277
    | Leu Lys Thr Val Leu Lys Cys His Ile Pro Asp Pro Ser Glu Phe Phe Ser Gln Leu Ser Ser Gln His Gly Gly |

276 | GAC CTT CAG AAA TGG CTC TCC GTC CCT TTC TTC AGC CCC AGT GGC CCT GCC CCT GAG ATC TCT | 1352
    | Asp Leu Gln Lys Trp Leu Ser Val Pro Phe Phe Ser Pro Ser Gly Pro Ala Pro Glu Ile Ser |

301 | CCG CTG GAA GTG CTC GAC GGA GAT TCC AAG TCT CTG CTG CTC CAG GTG GCC TCT GAC AAG GAC TCT CAG TTA CCC | 1427
    | Pro Leu Glu Val Leu Asp Gly Asp Ser Lys Ser Leu Leu Leu Gln Val Ala Ser Asp Lys Gln Leu Pro |

326 | TCG CCC AGC GGC CAC TCA CAG GCC AGC GCC TTC TTC ACC AAC CAG GGC TTC CAT CTG CCC AAT GCC TTG | 1502
    | Ser Pro Ser Gly His Ser Gln Ala Ser Gln Ala Ser Phe Phe Thr Asn Gln Gly Phe His Leu Pro Asn Ala Leu |

351 | GAG ATC TCC TGC CAG GTG TAC TTC TAC GTG GTG GAG GAG GAG GAT GGG GAG TCA AGG | 1577
    | Glu Ile Ser Cys Gln Val Tyr Phe Thr Tyr Asp Val Glu Glu Glu Asp Gly Asp Ser Arg |

376 | CTG CCC GAG GGA TCT CCT CAC CCA CTG TTC TCC TAC TGT TGC CCG CCC | 1652
    | Leu Pro Glu Gly Ser Pro His Pro Leu Phe Cys Tyr Asp Asp Phe Ala Pro Pro |

401 | AGG GAT GAC CTG CTG CTC TTC TCC CCG AGC AGC CTC TAT GGG CCT GAA | 1727
    | Arg Asp Asp Leu Leu Leu Phe Ser Pro Ser Leu Ala Tyr Gly Gly Ser Arg Ala Pro Glu |

426 | GAA AGA TCT CCA CTC TCC CAT GGA CTT GGA TCC CTA GCA GGG ATG TTA CAG CGC CCT | 1802
    | Glu Arg Ser Pro Leu Ser His Leu Gly Gly Leu Ser Ala Gly Met Leu Gln Arg Pro |

451 | CTG GAG ATG CCG GAA GGT GAG GAG TCT GCC CTG GGG GAG GCC AGT GTC CCA GAA | 1877
    | Leu Glu Met Pro Glu Gly Glu Glu Ser Ala Asn Asn Ser Gly Glu Ala Ser Val Pro Glu |

476 | GGC AAC CTT CAT CAA GAT CAG GAC AGA GGC CAG CCC ATC CTG ACC AAC ACC GAT GCC TAT CTG TCT | 1952
    | Gly Asn Leu His Gln Asp Gln Asp Arg Gly Gln Gly Pro Ile Leu Thr Asn Thr Asp Ala Tyr Leu Ser |

501 | CTT CAA GAA CTA CAG GCC CAA GAT TCA GTC CAC CTA ATA TAG | 2036
    | Leu Gln Glu Leu Gln Ala Gln Asp Ser Val His Leu Ile *** |

GCTTGAGGAAGACTGCTTAAGAGGTCTTCTTGAGGACAGCAGTGTCCTGGCTATCCCTGCCCCCCCCCTCCAAACTTAATCATCCACT | 2135
TCTGAACTCCATTTGCTACTTCCTGGTCTAACCAGGTTTGGTGGAGGGTGGGGAGCGGGGTCAGCTCCACTGCCCTATTTAGTCATGAG | 2234
GTCACTAGCTTCCCTGTACCTGCCTCCTTCCCTCCTTCACAGGCAGGGCAGCTAGAAACTTGCTTCCCCG | 2306

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 900 168  (THE UNITED STATES OF AMERICA)<br>* Page 6, line 1 - page 8, line 4; page 8, lines 5-30 * | 12-15 | C 12 N  15/12<br>C 12 P  21/02<br>C 12 N  5/20<br>C 12 P  21/08 //<br>(C 12 P  21/08<br>C 12 R  1:91 ) |
| Y | | 1-11 | |
| Y | EP-A-0 162 699  (IMMUNEX CORP.) | 1-11 | |
| T | SCIENCE, vol. 244, 5th May 1989, Washington, D.C., US; M. HATAKEYAMA et al.: "Interleukin-2 receptor beta chain gene: Generation of three receptor forms by cloned human alpha and beta chain cDNA's", pages 551-556<br>* Whole document * | 1-15 | |
| T,D | PROCEEDINGS OF THE NATIONAL ACADEMY OF SCIENCE, USA, vol. 86, March 1989, Washington DC., USA; M. TSUDO et al: "Characterization of the interleukin 2 receptor beta chain using three distinct monoclonal antibodies"<br>* Materials and methods * | 12-15 | |
| A | JOURNAL OF EXPERIMENTAL MEDICINE, January 1987, vol. 165, New York, N.Y., US; K. TESHIGAWARA et al.: "Interleukin 2 high-affinity receptor expression requires two distict binding proteins", pages 223-238<br>* Page 223, lines 6-9 * | | **TECHNICAL FIELDS SEARCHED (Int. Cl.5)**<br><br>C 12 N<br>C 12 P |

The present search report has been drawn up for all claims

| Place of search | Date of completion of the search | Examiner |
|---|---|---|
| THE HAGUE | 21-08-1989 | VAN PUTTEN A.J. |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P0401)